# EUROPEAN PATENT APPLICATION

(11) **EP 4 071 182 A1**
(43) Date of publication of application: **12.10.2022**
(21) Application number: 20895495.8
(22) Date of filing: 01.12.2020
(51) Int. Cl.: C08F 20/00, C08G 59/20, C08G 65/14, C07D 303/44, C07C 69/54

(54) **ALICYCLIC ACRYLATE COMPOUND, ALICYCLIC EPOXY ACRYLATE COMPOUND, CURABLE COMPOSITION, AND CURED ARTICLE**

(30) Priority: 02.12.2019 JP 2019218298
(71) Applicant: ENEOS Corporation, Chiyoda-ku Tokyo 100-8162 (JP)
(72) Inventor: UE, Kenta, Tokyo 100-8162 (JP); KOIKE, Takeshi, Tokyo 100-8162 (JP); KAMEYAMA, Atsushi, Tokyo 100-8162 (JP)
(74) Representative: Müller-Boré & Partner Patentanwälte PartG mbB
(86) International application number: PCT/JP2020/044604
(87) International publication number: WO 2021/112062

(57) **Abstract**

The present invention provides an alicyclic epoxy acrylate compound that can have high heat resistance in the form of a cured product when contained in a curable composition, a curable composition, and a cured product.
An alicyclic epoxy acrylate compound represented by the following Formula (1) where A denotes an oxygen atom and a curable composition comprising thereof are used: 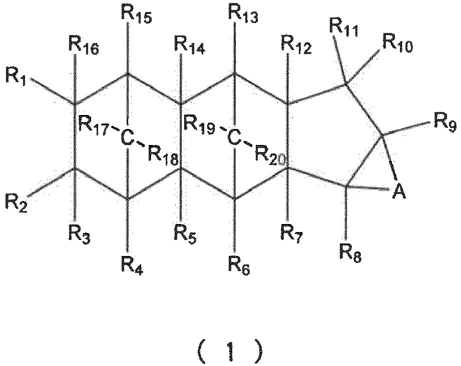 wherein one of R₁ and R₂ is a (meth)acryloyloxy group, the other of R₁ and R₂ is a hydrogen atom, R₃ to R₂₀ are each independently selected from the group consisting of a hydrogen atom, an alkyl group, and an alkoxy group, and A is an oxygen atom or A is not present, and a carbon atom to which R₈ binds and a carbon atom to which R₉ binds together form a double bond.

## Description

### CROSS-REFERENCE TO RELATED APPLICATIONS

The present patent application is based upon and claims the benefit of priority from Japanese Patent Application No. 2019-218298 (filed on December 2, 2019). The entire disclosures of the above described patent applications are incorporated herein by reference.

### BACKGROUND OF THE INVENTION

### Field of the Invention

The present invention relates to an alicyclic acrylate compound, an alicyclic epoxy acrylate compound, a curable composition, and a cured product.

### Background Art

An epoxy group-containing (meth)acrylic compound and an acrylate having an alicyclic skeleton are generally a compound that is useful in the fields of resists, sealing materials, coatings, inks, adhesives, sealants, and the like. Here, an acrylate containing an epoxy group with an alicyclic skeleton is known to have properties suitable for outdoor use. They generally exert a certain performance by the ring opening reaction of the oxirane ring present in the structure, the reaction of the ethylene-based unsaturated group of the acrylate, or the like.

For example, Patent Document 1 discloses that a vinyl group-containing alicyclic acrylate compound having a specific alicyclic skeleton is particularly useful for coating.

Further, Patent Document 2 discloses, as a resin composition containing an epoxy group-containing (meth)acrylic compound, a cured product of a resin composition containing a specific epoxy group-containing (meth)acrylate that is excellent in heat resistance and the like.

However, conventional epoxy group-containing (meth)acrylic compounds as suggested in Patent Documents 1 and 2 had room for further improvement in terms of heat resistance of cured products.

### Prior Art Documents

### Patent Documents

Patent Document 1: JP H11-209326 A
Patent Document 2: JP 2006-131866 A

### SUMMARY OF THE INVENTION

### Problems to be Solved by the Invention

In applications such as resists, sealing materials, and coatings, there is still a cured product having further improved high heat resistance such that it can be adapted to more severe use conditions.

Therefore, an object of the present invention is to provide an alicyclic epoxy acrylate compound that can have high heat resistance in the form of a cured product when contained in a curable composition. Another object of the present invention is to provide an epoxy group-containing (meth)acrylic polymer and a radical-polymerizable compound, which can have high heat resistance in the form of a cured product when contained in a curable composition. Yet another object of the present invention is to provide a curable composition for obtaining a cured product having high heat resistance.

### Means for Solving the Problems

[1] An alicyclic acrylate compound represented by the following Formula (1): 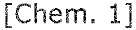 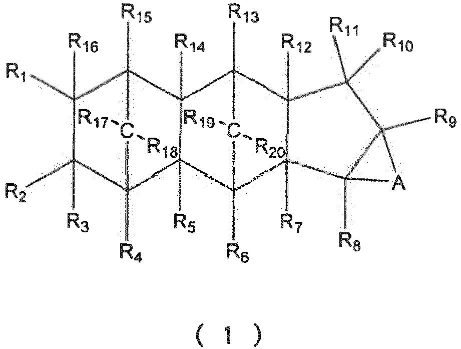 wherein one of R₁ and R₂ is a (meth)acryloyloxy group,
   the other of R₁ and R₂ is a hydrogen atom,
   R₃ to R₂₀ are each independently selected from the group consisting of a hydrogen atom, an alkyl group, and an alkoxy group, and
   A is an oxygen atom or A is not present, and a carbon atom to which R₈ binds and a carbon atom to which R₉ binds together form a double bond.
[2] An alicyclic acrylate-type polymerizable monomer comprising the alicyclic acrylate compound according to [1].
[3] The alicyclic acrylate compound according to [1], which is an alicyclic epoxy acrylate compound represented by the Formula (1), wherein A is an oxygen atom.
[4] An alicyclic epoxy acrylate-type polymerizable monomer comprising the alicyclic epoxy acrylate compound according to [3].
[5] A curable composition comprising at least the alicyclic epoxy acrylate-type polymerizable monomer compound according to [4].
[6] The curable composition according to [5], which further comprises at least one selected from the group consisting of a curing agent, a thermal cationic polymerization initiator, and a photo-cationic polymerization initiator.
[7] The curable composition according to [6], wherein the curing agent is an acid anhydride-based compound.
[8] The curable composition according to any one of [5] to [7], which further comprises a thermal radical polymerization initiator.
[9] The curable composition according to any one of [5] to [8], which further comprises an epoxy compound that is different from the alicyclic epoxy acrylate compound.
[10] The curable composition according to any one of [5] to [9], which further comprises a radical-polymerizable compound that is different from the alicyclic epoxy acrylate compound.
[11] An epoxy group-containing (meth)acrylic polymer comprising at least a polymerization unit represented by the following Formula (2): 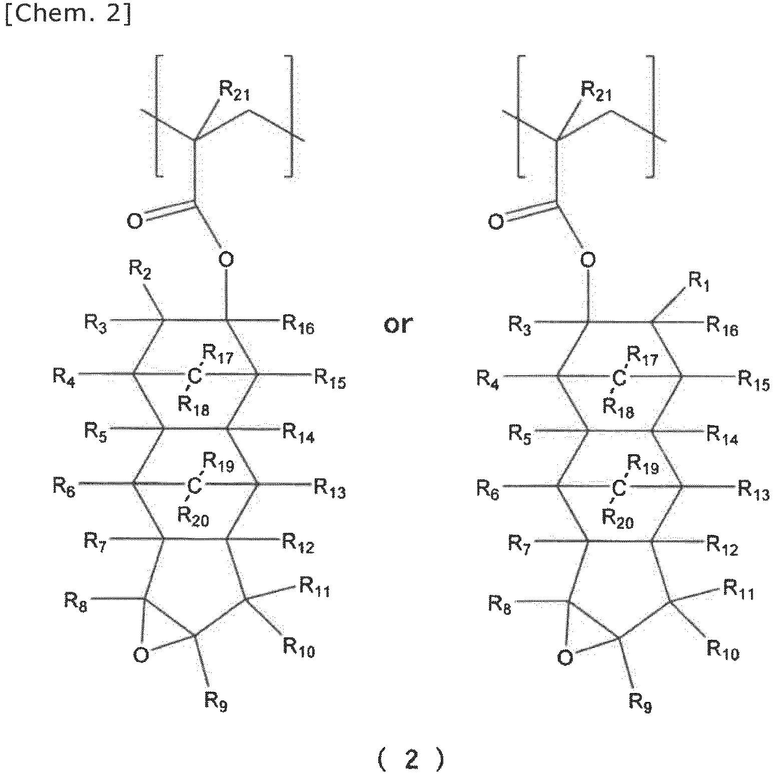 wherein R₁ and R₂ are each a hydrogen atom,
   R₃ to R₂₀ are each independently selected from the group consisting of a hydrogen atom, an alkyl group, and an alkoxy group, and
   R₂₁ is a hydrogen atom or a methyl group.
[12] A curable composition comprising at least the epoxy group-containing (meth)acrylic polymer according to [11].
[13] The curable composition according to [12], which further comprises a photo-cationic polymerization initiator.
[14] The curable composition according to [12] or [13], which further comprises an epoxy compound that is different from the epoxy group-containing (meth)acrylic polymer.
[15] A radical-polymerizable compound comprising at least a polymerization unit represented by the following Formula (3): 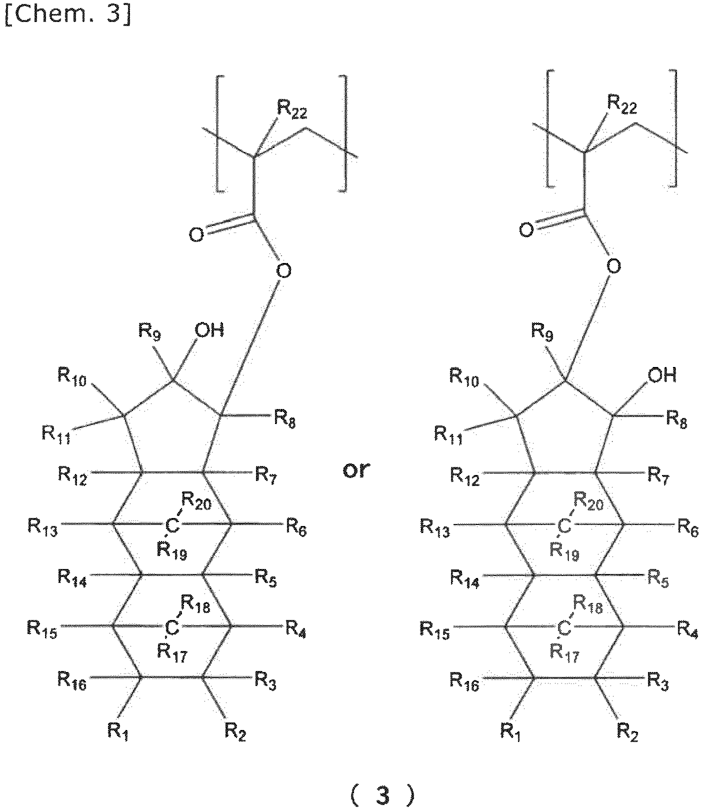 wherein one of R₁ and R₂ is a (meth)acryloyloxy group,
   the other of R₁ and R₂ is a hydrogen atom,
   R₃ to R₂₀ are each independently selected from the group consisting of a hydrogen atom, an alkyl group, and an alkoxy group, and
   R₂₂ is selected from a hydrogen atom and a methyl group.
[16] The radical-polymerizable compound according to [15], which further comprises a polymerization unit derived from at least one acrylic compound selected from (meth)acrylic acid ester and (meth)acrylic acid.
[17] A curable composition comprising at least the radical-polymerizable compound according to [15] or [16].
[18] The curable composition according to [17], which further comprises a photoradical polymerization initiator.
[19] The curable composition according to [17] or [18], which further comprises a radical-polymerizable compound that is different from the radical-polymerizable compound.
[20] A method of producing an epoxy group-containing acrylic polymer, comprising a step of radically polymerizing the alicyclic epoxy acrylate compound according to [3].
[21] A method of producing a radical-polymerizable compound, comprising a step of performing radical polymerization of a polymer of at least one acrylic compound selected from (meth)acrylic acid ester and (meth)acrylic acid with the alicyclic epoxy acrylate compound according to [3].

### Effect of the Invention

According to the present invention, an alicyclic epoxy acrylate compound that can have high heat resistance in the form of a cured product when contained in a curable composition can be provided. In addition, according to the present invention, an epoxy group-containing (meth)acrylic polymer and a radical-polymerizable compound, which can have high heat resistance in the form of a cured product when contained in a curable composition can be provided. Further, according to the present invention, a curable composition for obtaining a cured product having high heat resistance can be provided.

### BRIEF DESCRIPTION OF THE DRAWINGS

FIG. 1 shows an ¹H-NMR chart of the alicyclic acrylate-type polymerizable monomer synthesized in Preparation Example 1-1.
FIG. 2 shows a ¹³C-NMR chart of the alicyclic acrylate-type polymerizable monomer synthesized in Preparation Example 1-1.
FIG. 3 shows an ¹H-NMR chart of the alicyclic epoxy acrylate-type polymerizable monomer (A1) synthesized in Preparation Example 1-2.
FIG. 4 shows a ¹³C-NMR chart of the alicyclic epoxy acrylate-type polymerizable monomer (A1) synthesized in Preparation Example 1-2.
FIG. 5 shows an infrared absorption spectrum (IR) of the epoxy group-containing (meth)acrylic polymer obtained in Example 3-1.
FIG. 6 shows an infrared absorption spectrum (IR) of the radical-polymerizable compound obtained in Example 4-1.

### DETAILED DESCRIPTION OF THE INVENTION

### Definitions

In the present specification, the terms "part(s)," "%" and the like used to describe the composition are represented on a mass basis, unless otherwise specified.

### Alicyclic Acrylate Compound

According to one embodiment of the present invention, the alicyclic acrylate compound is an alicyclic acrylate compound represented by the following Formula (1). A curable composition, which contains an alicyclic epoxy acrylate compound for which A is an oxygen atom in the following Formula (1) for the alicyclic acrylate compound, can be provided. In addition, an alicyclic acrylate compound of the following Formula (1) where A is not present and the carbon atom to which R₈ binds and the carbon atom to which R₉ binds together form a double bond can be used as a starting material for an alicyclic epoxy acrylate compound. 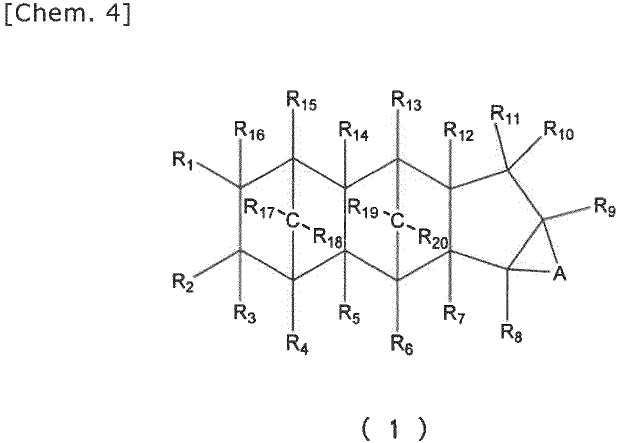 wherein, one of R₁ and R₂ is a (meth)acryloyloxy group,
the other of R₁ and R₂ is a hydrogen atom,
R₃ to R₂₀ are each independently selected from the group consisting of a hydrogen atom, an alkyl group, and an alkoxy group, and
A is an oxygen atom or A is not present, and a carbon atom to which R₈ binds and a carbon atom to which R₉ binds together form a double bond.

According to one embodiment of the present invention, an alicyclic acrylate-type polymerizable monomer is provided. Here, the alicyclic acrylate-type polymerizable monomer may be a single alicyclic acrylate compound or a mixture of alicyclic acrylate compounds. The polymerizable monomer may be, for example, one or more kinds of alicyclic acrylate compounds, a combination of two or more kinds of alicyclic acrylate compounds, or a combination of two kinds of alicyclic acrylate compounds.

In the alicyclic acrylate compound represented by the following Formula (1), one of R₁ and R₂ is a (meth)acryloyloxy group, the other of R₁ and R₂ is a hydrogen atom, preferably, one of R₁ and R₂ is a (meth)acryloyloxy group, and the other of R₁ and R₂ is a hydrogen.

According to another embodiment of the present invention, in the alicyclic acrylate compound represented by Formula (1), R₁ is a (meth)acryloyloxy group, R₂ is a hydrogen atom or R₁ is a hydrogen atom, R₂ is a (meth)acryloyloxy group, R₁ is preferably a methacryloyloxy group, R₂ is a hydrogen atom or R₁ is a hydrogen atom, and R₂ is a methacryloyloxy group.

In the alicyclic acrylate compound represented by the following Formula (1), R₃ to R₂₀ are each independently selected from the group consisting of a hydrogen atom, an alkyl group, and an alkoxy group, preferably selected from the group consisting of a hydrogen atom and an alkyl group, and more preferably a hydrogen atom.

In the alicyclic acrylate compound represented by Formula (1), preferably, R₃ and R₁₆ are each a hydrogen atom,

The number of carbon atoms in alkyl groups of R₃ to R₂₀ in the above-described Formula (1) is, but is not particularly limited to, preferably from 1 to 10 and more preferably from 1 to 5. The above-described alkyl groups may be a linear alkyl group or branched alkyl group.

Examples of the linear alkyl group include a methyl group, an ethyl group, an n-propyl group, and an n-butyl group.

Examples of the branched alkyl group include an isopropyl group, an isobutyl group, and a t-butyl group.

The number of carbon atoms in alkoxy groups of R₃ to R₂₀ in the above-described Formula (1) is, but is not particularly limited to, preferably from 1 to 10 and more preferably from 1 to 5.

Examples of an alkoxy group include a methoxy group, an ethoxy group, a propyloxy group, an i-propyloxy group, an n-butoxy group, an i-butoxy group, an s-butoxy group, and a t-butoxy group.

According to a preferred embodiment of the present invention, the alicyclic acrylate-type polymerizable monomer comprising the alicyclic acrylate compound represented by Formula (1) comprises a compound (1-i) represented by Formula (1) where R₁ is a (meth)acryloyloxy group and R₂ is a hydrogen atom, a compound (1-ii) represented by Formula (1) where R₂ is a (meth)acryloyloxy group and R₁ is a hydrogen atom, or a mixture thereof. Hereinafter, an alicyclic acrylate-type polymerizable monomer comprising an alicyclic (epoxy)acrylate compound represented by Formula (X) is referred to as an "(epoxy)acrylate-type polymerizable monomer represented by Formula (X)."

According to a preferred embodiment of the present invention, the alicyclic acrylate-type polymerizable monomer comprising the alicyclic acrylate compound represented by Formula (1) comprises a compound (1-i-1) represented by Formula (1) where R₁ is a (meth)acryloyloxy group and R₂ to R₂₀ are each a hydrogen atom, a compound (1-ii-1) represented by Formula (1) where R₂ is a (meth)acryloyloxy group and R₁ and R₃ to R₂₀ are each a hydrogen atom, or a mixture thereof.

According to one embodiment of the present invention, an alicyclic acrylate compound for which A is not present and the carbon atom to which R₈ binds and the carbon atom to which R₉ binds together form a double bond in Formula (1) above is represented by the following Formula (1-1). 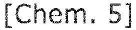 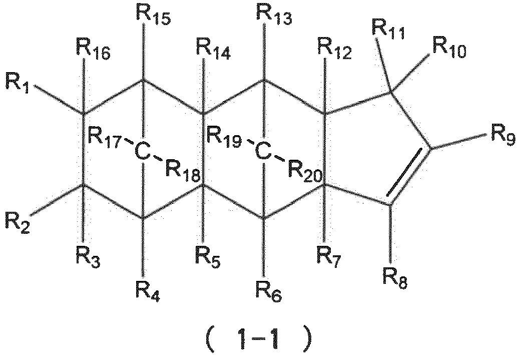 wherein, R₁ to R₂₀ are as shown in Formula (1).

According to one embodiment of the present invention, an alicyclic acrylate-type polymerizable monomer comprising an alicyclic acrylate compound represented by Formula (1-1) (hereinafter, also referred to as "alicyclic acrylate-type polymerizable monomer represented by Formula (1-1)) is provided. Here, the alicyclic acrylate-type polymerizable monomer may be a single alicyclic acrylate compound or a mixture of alicyclic acrylate compounds. The alicyclic acrylate-type polymerizable monomer may be, for example, one or more kinds of alicyclic acrylate compounds, a combination of two or more kinds of alicyclic acrylate compounds, or a combination of two kinds of alicyclic acrylate compounds.

According to a preferred embodiment of the present invention, the alicyclic acrylate-type polymerizable monomer comprising the alicyclic acrylate compound represented by Formula (1-1) comprises a compound (1-1-i) represented by Formula (1-1) where R₁ is a (meth)acryloyloxy group and R₂ is a hydrogen atom, a compound (1-1-ii) represented by Formula (1-1) where R₂ is a (meth)acryloyloxy group and R₁ is a hydrogen atom, or a mixture thereof.

According to a preferred embodiment of the present invention, the alicyclic acrylate-type polymerizable monomer comprising the alicyclic acrylate compound represented by Formula (1-1) comprises a compound in which one of R₁ and R₂ is a methacryloyloxy group and the other of R₁ and R₂ and R₃ to R₂₀ are each a hydrogen atom. A more preferred embodiment of the above is represented by the following Formula (1-1-i-1). Specifically, Formula (1-1-i-1) shows that any one of groups corresponding to R₁ and R₂ bound to the norbornane skeleton in Formula (1) is a methacryloyloxy group.

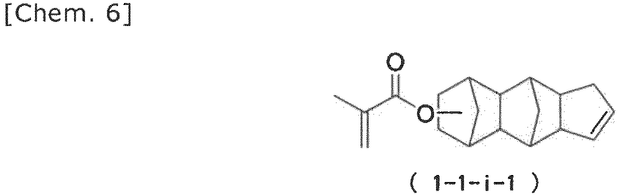

Therefore, the alicyclic acrylate-type polymerizable monomer represented by Formula (1-1-i-1) is a compound represented by the following Formula (1-1-i-2), a compound represented by the following Formula (1-1-i-3), or a mixture thereof (hereinafter, a compound represented by Formula (X) is also referred to as "compound (X)"). The alicyclic acrylate-type polymerizable monomer represented by Formula (1-1-i-1) is preferably a compound represented by Formula (1-1-i-3).

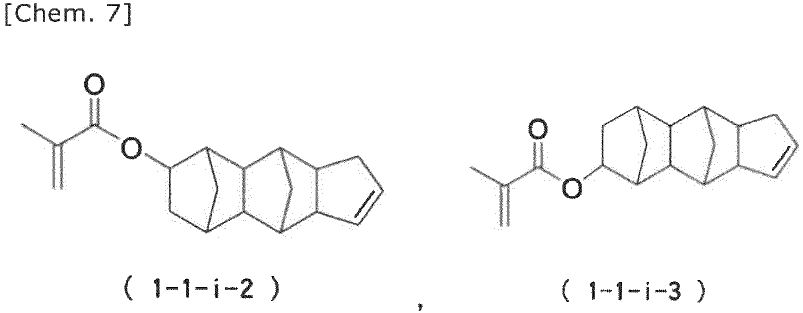

According to one embodiment of the present invention, an alicyclic acrylate compound of the above-described Formula (1) where A is an oxygen atom is an alicyclic epoxy acrylate compound having an epoxy group which is represented by the following Formula (1-2). 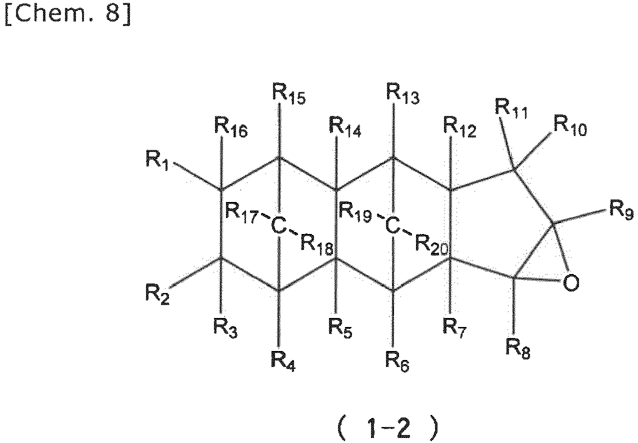 [In the formula, R₁ to R₂₀ are as shown in Formula (1).]

According to one embodiment of the present invention, an alicyclic epoxy acrylate-type polymerizable monomer comprising an alicyclic epoxy acrylate compound represented by Formula (1-2) (hereinafter, also referred to as "alicyclic epoxy acrylate-type polymerizable monomer represented by Formula (1-2)) is provided. Here, the alicyclic epoxy acrylate-type polymerizable monomer may be a single alicyclic epoxy acrylate compound or a mixture of alicyclic epoxy acrylate compounds. The alicyclic epoxy acrylate-type polymerizable monomer may be, for example, one or more kinds of alicyclic epoxy acrylate compounds, a combination of two or more kinds of alicyclic epoxy acrylate compounds, or a combination of two kinds of alicyclic epoxy acrylate compounds.

According to a preferred embodiment of the present invention, the alicyclic epoxy acrylate-type polymerizable monomer comprising the alicyclic epoxy acrylate compound represented by Formula (1-2) comprises a compound (1-2-i) represented by Formula (1-2) where R₁ is a (meth)acryloyloxy group and R₂ is a hydrogen atom, a compound (1-2-ii) represented by Formula (1-2) where R₂ is a (meth)acryloyloxy group and R₁ is a hydrogen atom, or a mixture thereof.

According to a preferred embodiment of the present invention, in the alicyclic epoxy acrylate-type polymerizable monomer comprising the alicyclic epoxy acrylate compound represented by Formula (1-2), one of R₁ and R₂ is a methacryloyloxy group and the other of R₁ and R₂ and R₃ to R₂₀ are each a hydrogen atom. A more preferred embodiment of the above is represented by the following Formula (1-2-i-1). Specifically, Formula (1-2-i-1) shows that any one of groups corresponding to R₁ and R₂ bound to the norbornane skeleton in Formula (1) is a methacryloyloxy group.

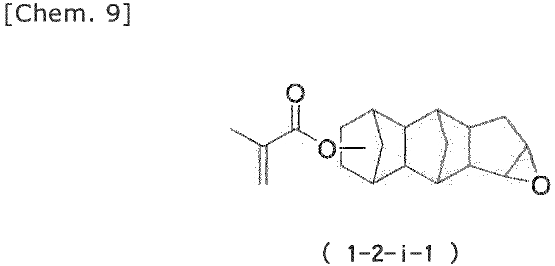

Therefore, the alicyclic epoxy acrylate-type polymerizable monomer represented by Formula (1-2-i-1) is a compound represented by the following Formula (1-2-i-2), a compound represented by the following Formula (1-2-i-3), or a mixture thereof. The alicyclic epoxy acrylate-type polymerizable monomer represented by Formula (1-2-i-1) is preferably a compound represented by Formula (1-2-i-3).

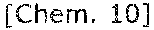

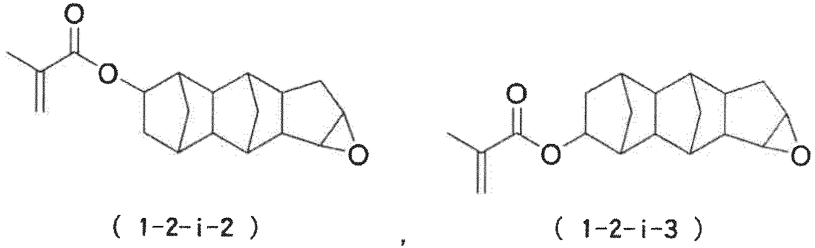

### Method of Producing Alicyclic Acrylate Compound (Compound of Formula (1-1)1

The above-described alicyclic acrylate compound represented by Formula (1-1) can be obtained by a method comprising a step of reacting a compound represented by the following Formula (1-a) optionally with an acrylic compound. 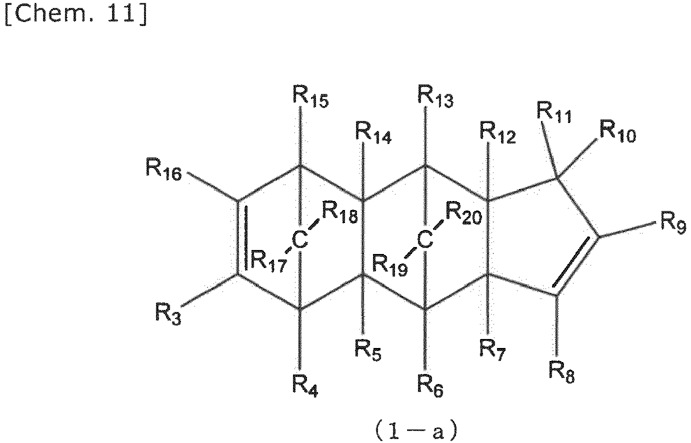 [In the formula, R₃ to R₂₀ are each independently selected from the group consisting of a hydrogen atom, an alkyl group, and an alkoxy group.]

According to one embodiment of the present invention, the method of producing an alicyclic acrylate compound comprises a step of reacting the above-described compound represented by Formula (1-a) with an acrylic compound.

According to one embodiment of the present invention, in the production of an alicyclic acrylate compound, it is preferable to perform purification by distillation, a column, or recrystallization in cases where the purity of an alicyclic acrylate compound obtained by the above-described production method is low.

According to one embodiment of the present invention, examples of an acrylic compound that can be used for the production of an alicyclic acrylate compound include (meth)acrylic acid and preferably methacrylic acid. One kind of acrylic compound or, as necessary, a combination of two or more kinds thereof may be used.

According to one embodiment of the present invention, the amount of the acrylic compound used in the production of the alicyclic acrylate compound is, but is not particularly limited to, preferably from 0.10 to 1.80 mol and more preferably from 0.50 to 1.50 mol with respect to 1.00 mol of the compound represented by Formula (1-a).

Examples of the compound represented by Formula (1-a) include a compound of Formula (1-a-1), which can be obtained by allowing cyclopentadiene and dicyclopentadiene to undergo the Diels-Alder reaction.

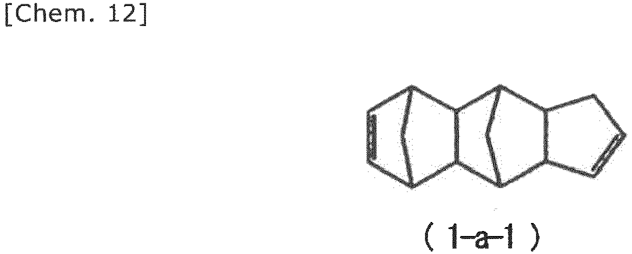

According to one embodiment of the present invention, it is preferable to produce an alicyclic acrylate compound in the presence of an acid as appropriate. Examples of an acid that can be used for the production of an alicyclic acrylate compound include various acids such as inorganic acids and organic acids and combinations thereof. Specifically, as inorganic acids, iron (III) chloride, Lewis acid such as boron trifluoride, sulfuric acid, hydrogen chloride, hydrochloric acid, phosphoric acid, heteropolyacids, zeolites, clay minerals, and the like can be exemplified, and as organic acids, trifluoromethanesulfonic acid, methanesulfonic acid, paratoluenesulfonic acid, ion exchange resin, and the like or combinations thereof can be exemplified.

The amount of an acid used is usually from 0.001 to 1 mol, preferably from 0.005 to 0.5 mol, and more preferably from 0.01 to 0.1 mol with respect to 1 mol of the compound represented by Formula (1-a). Here, in cases where the acid is a combination of a plurality of acids, the amount of the acid used means the total thereof.

### Starting Material Production Step (Method of Producing Alicyclic Epoxy Acrylate Compound (Compound of Formula (1-2)))

According to one embodiment of the present invention, the above-described alicyclic epoxy acrylate compound represented by Formula (1-2) can be obtained by a method comprising a step of reacting the above-described alicyclic acrylate compound represented by Formula (1-1) with a peracid.

According to one embodiment of the present invention, in the production of an alicyclic epoxy acrylate compound of Formula (1-2), it is preferable to perform purification by distillation, a column, or recrystallization in cases where the purity of an alicyclic epoxy acrylate compound of Formula (1-2) obtained by the above-described production method is low.

According to one embodiment of the present invention, examples of a peracid that can be used in the production of an alicyclic epoxy acrylate compound of Formula (1-2) include organic peracids such as performic acid, peracetic acid, perbenzoic acid, meta-chloroperbenzoic acid, and trifluoroperacetic acid, and hydrogen peroxide. Of these, meta-chloroperbenzoic acid and hydrogen peroxide are preferred because of their excellent availability and reactivity.

The amount of the peracid used in the production of an alicyclic epoxy acrylate compound of Formula (1-2) is preferably from 0.10 to 2.50 mol and more preferably from 0.50 to 1.80 mol with respect to 1.00 mol of the above-described alicyclic acrylate compound represented by Formula (1-1).

### Usefulness of Alicyclic Epoxy Acrylate Compound

According to one embodiment of the present invention, when allowing the curable composition to contain the alicyclic epoxy acrylate compound, a cured product of the curable composition can have high heat resistance. Therefore, the alicyclic epoxy acrylate compound can be appropriately used in various fields involving various coatings of cans, plastics, papers, woods, and the like, inks, adhesives, sealants, resists, sealings, and the like.

More specifically, the alicyclic epoxy acrylate compound can be appropriately used for 3D modeling materials, acid removers, furniture coatings, decorative coatings, car undercoatings, finish coatings, coatings of beverage cans and other cans, UV curable inks, protective films for optical disc recording layer, color filter protective films, adhesives for bonding optical discs, adhesives for optical materials, die bonding of semiconductor devices, sealing materials for organic EL displays, CCD, sealants for light receiving devices such as infrared sensors, sealants for light emitting devices such as LEDs and organic EL, optical wiring boards, optical connectors, optical semiconductor related parts such as lenses, optical waveguides, photoresists, and composite glass such as tempered glass and security glass. It is also useful as a monomer constituting a polymer and a precursor of a silane coupling agent.

### Epoxy Group-Containing (Meth)acrylic Polymer

According to one embodiment of the present invention, the epoxy group-containing (meth)acrylic polymer is an epoxy group-containing (meth)acrylic polymer comprising at least a polymerization unit represented by the following Formula (2). 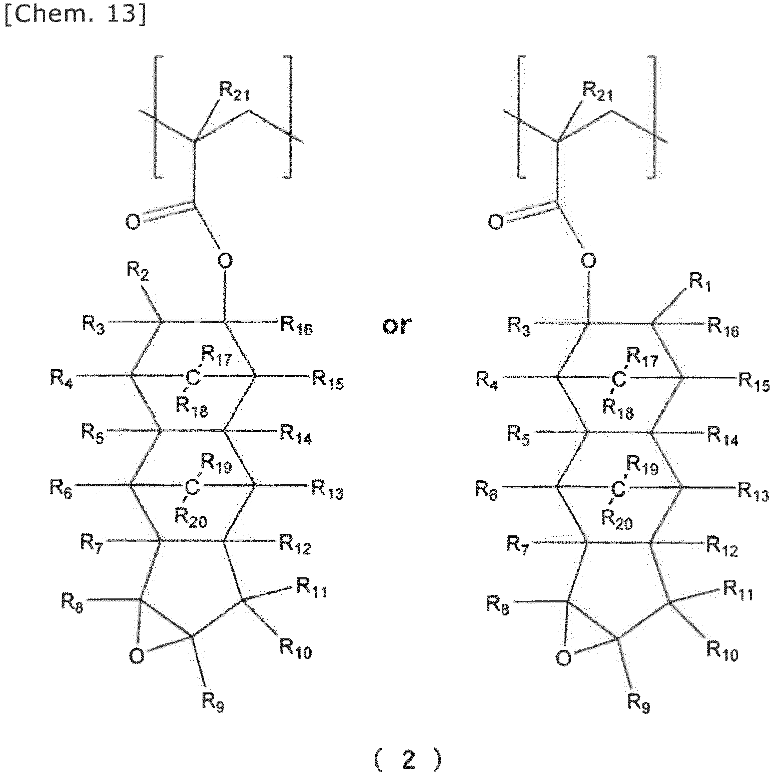 wherein, R₁ and R₂ are each a hydrogen atom,
R₃ to R₂₀ are each independently selected from the group consisting of a hydrogen atom, an alkyl group, and an alkoxy group, and
R₂₁ is a hydrogen atom or a methyl group.

In an epoxy group-containing (meth)acrylic polymer comprising the polymerization unit represented by Formula (2), R₃ to R₂₀ are each independently selected from the group consisting of a hydrogen atom, an alkyl group, and an alkoxy group, preferably selected from a hydrogen atom and an alkyl group, and more preferably a hydrogen atom.

Preferably, R₃ and R₁₆ are each a hydrogen atom in Formula (2).

The number of carbon atoms in alkyl groups of R₃ to R₂₀ in the above-described Formula (2) is, but is not particularly limited to, preferably from 1 to 10 and more preferably from 1 to 5. The above-described alkyl groups may be a linear alkyl group or branched alkyl group.

Examples of the linear alkyl group include a methyl group, an ethyl group, an n-propyl group, and an n-butyl group.

Examples of the branched alkyl group include an isopropyl group, an isobutyl group, and a t-butyl group.

The number of carbon atoms in alkoxy groups of R₃ to R₂₀ in the above-described Formula (2) is, but is not particularly limited to, preferably from 1 to 10 and more preferably from 1 to 5.

The alkyl groups as part of the above-described alkoxy groups may be a linear alkyl group or branched alkyl group.

Examples of an alkoxy group include a methoxy group, an ethoxy group, a propyloxy group, an i-propyloxy group, an n-butoxy group, an i-butoxy group, an s-butoxy group, and a t-butoxy group.

R₂₁ in Formula (2) above is a hydrogen atom or a methyl group and preferably a methyl group.

According to a preferred embodiment of the present invention, the epoxy group-containing (meth)acrylic polymer comprises a polymerization unit represented by the following Formula (2-1), a polymerization unit represented by the following Formula (2-2), or a combination thereof. Therefore, the polymerization unit represented by Formula (2) may be a polymerization unit represented by the following Formula (2-1), a polymerization unit represented by the following Formula (2-2), or a combination thereof. Here, in Formula (2-1) and Formula (2-2) above, R₁ to R₂₁ may be different from one another.

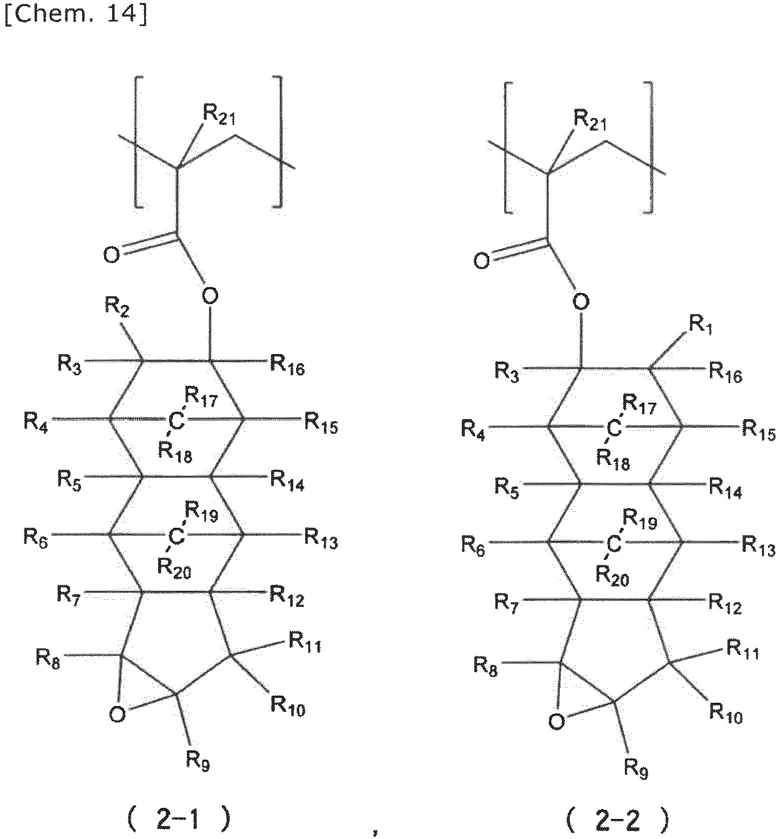

According to a preferred embodiment of the present invention, R₃ to R₂₀ are each a hydrogen atom and R₂₁ is a methyl group in Formula (2) above. A more preferred embodiment of the above is represented by the following Formula (2-3). Note that in Formula (2-3), the position of the bond between the methacryloyloxy group and the norbornane skeleton is not specified, but the methacryloyloxy group-derived oxygen atom is assumed to be bound to the norbornane skeleton at the position corresponding to either R₁ or R₂ in Formula (1).

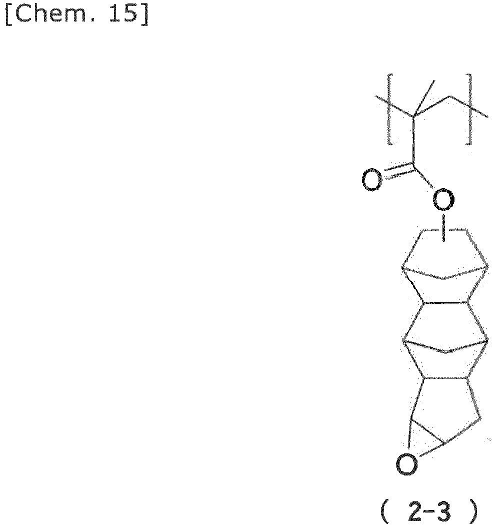

According to one embodiment of the present invention, the polymerization unit represented by Formula (2-3) is a polymerization unit represented by the following Formula (2-4), a polymerization unit represented by the following Formula (2-5), or a combination thereof.

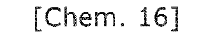

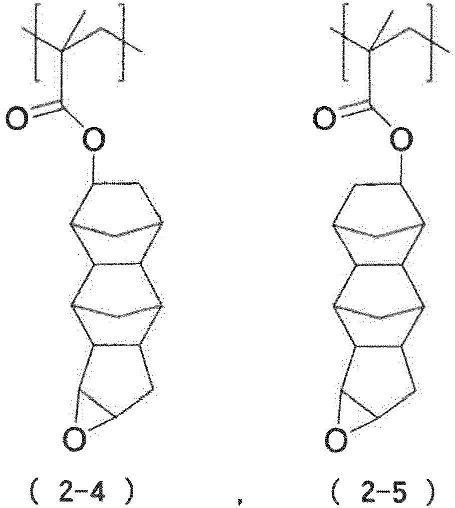

According to one embodiment of the present invention, the epoxy group-containing (meth)acrylic polymer comprises a polymerization unit represented by Formula (2-4), a polymerization unit represented by the following Formula (2-5), or a combination thereof.

According to a more preferred embodiment of the present invention, the above-described polymerization unit represented by Formula (2-3) is the polymerization unit represented by Formula (2-4).

Note that, according to one embodiment of the present invention, the above-described polymerization unit represented by Formula (2) may be a combination of polymerization units in which R₁ to R₂₁ are different from one another. The present invention also encompasses such an aspect.

According to one embodiment of the present invention, the content of the polymerization unit represented by Formula (2) in the epoxy group-containing (meth)acrylic polymer is, for example, 80 mol% or more, preferably 90 mol% or more, and more preferably 95 mol% or more the total amount (100 mol%) of all polymerization units in the epoxy group-containing (meth)acrylic polymer. The upper limit thereof is not particularly limited, but it is, for example, 100 mol% or less.

According to one embodiment of the present invention, the number average molecular weight (Mn) of the epoxy group-containing (meth)acrylic polymer is, for example, 500 or more, and can be preferably from 1,000 to 200,000 and more preferably from 10,000 to 50,000. The number average molecular weight can be measured by size exclusion chromatography.

According to one embodiment of the present invention, the weight average molecular weight (Mw) of the epoxy group-containing (meth)acrylic polymer is, for example, 5,000 or more, and can be preferably from 10,000 to 200,000 and more preferably from 50,000 to 100,000. The weight average molecular weight can be measured by size exclusion chromatography.

### Method of Producing Epoxy Group-Containing (Meth)acrylic Polymer

According to one embodiment of the present invention, the epoxy group-containing (meth)acrylic polymer can be obtained by a method comprising a step of allowing the above-described alicyclic epoxy acrylate compound of Formula (1-2) to undergo radical polymerization.

According to one embodiment of the present invention, in radical polymerization, a radical polymerization method in a normal acrylic resin can be appropriately selected according to the common technical knowledge well known to those skilled in the art.

According to one embodiment of the present invention, in the method of producing an epoxy group-containing (meth)acrylic polymer, preferably, the epoxy group-containing (meth)acrylic polymer can be obtained by a method comprising a step of allowing the above-described alicyclic epoxy acrylate compound of Formula (1-2) to undergo radical polymerization in the presence of a photoradical polymerization initiator or a thermal radical polymerization initiator. In the above-described step, further, an epoxy compound which is different from the alicyclic epoxy acrylate compound of Formula (1-2) and/or the epoxy group-containing (meth)acrylic polymer comprising the polymerization unit represented by Formula (2) may be additionally mixed. By mixing such an epoxy compound, a mixture of an epoxy group-containing (meth)acrylic polymer and an epoxy compound can be obtained. Specific examples of the photoradical polymerization initiator and the thermal radical polymerization initiator described above include a photoradical polymerization initiator and a thermal radical polymerization initiator which are used for a curable composition described later.

According to one embodiment of the present invention, the amount of a photoradical polymerization initiator or a thermal radical polymerization initiator used in the production of an epoxy group-containing (meth)acrylic polymer is preferably from 0.1 to 100 mol% and more preferably from 1 to 30 mol% with respect to alicyclic epoxy acrylate compound of Formula (1-2).

In the case of using a thermal radical polymerization initiator as the radical polymerization initiator, it is preferable to perform agitation at 50°C to 150°C for 1 to 10 hours, for example, in the above-described radical polymerization step.

In the case of using a photoradical polymerization initiator as the radical polymerization initiator, the cumulative irradiation dose (accumulated amount of light) of an active energy ray may be, for example, from 100 to 10000 mJ/cm² and is preferably from 500 to 4000 mJ/cm² in the above-described radical polymerization step. Here, the cumulative irradiation dose (accumulated amount of light) refers to the irradiation dose expressed by the product of irradiation intensity and irradiation time of an active energy ray (e.g., visible ray, UV ray, X-ray, or electron beam)

### Usefulness of Epoxy Group-Containing (Meth)acrylic Polymer

According to one embodiment of the present invention, when allowing the curable composition to contain the epoxy group-containing (meth)acrylic polymer, a cured product of the curable composition can have high heat resistance. Therefore, it can be used as with the above-described alicyclic epoxy acrylate compound.

### Radical-polymerizable Compound

According to one embodiment of the present invention, the radical-polymerizable compound is a radical-polymerizable compound comprising at least a polymerization unit represented by the following Formula (3). 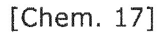 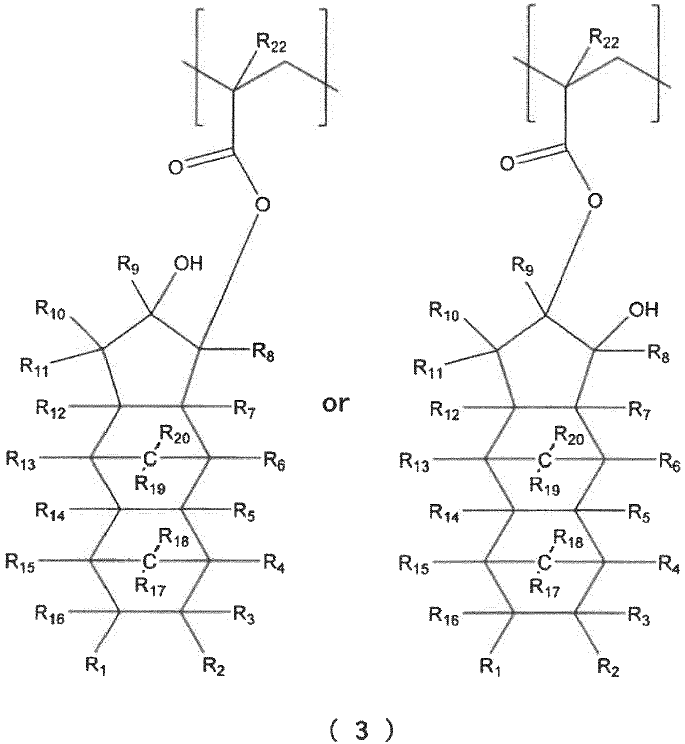 wherein, one of R₁ and R₂ is a (meth)acryloyloxy group,
the other of R₁ and R₂ is a hydrogen atom,
R₃ to R₂₀ are each independently selected from the group consisting of a hydrogen atom, an alkyl group, and an alkoxy group, and
R₂₂ is selected from a hydrogen atom and a methyl group.

In the polymerization unit represented by Formula (3), R₃ to R₂₀ are each independently selected from the group consisting of a hydrogen atom, an alkyl group, and an alkoxy group, preferably selected from the group consisting of a hydrogen atom and an alkyl group, and more preferably a hydrogen atom.

In the polymerization unit represented by Formula (3), preferably, R₃ and R₁₆ are each a hydrogen atom.

The number of carbon atoms in alkyl groups of R₃ to R₂₀ in the above-described Formula (3) is, but is not particularly limited to, preferably from 1 to 10 and more preferably from 1 to 5. The above-described alkyl groups may be a linear alkyl group or branched alkyl group.

Examples of the linear alkyl group include a methyl group, an ethyl group, a propyl group, and an n-butyl group.

Examples of the branched alkyl group include an isopropyl group, an isobutyl group, and a t-butyl group.

The number of carbon atoms in alkoxy groups of R₃ to R₂₀ in the above-described Formula (3) is, but is not particularly limited to, preferably from 1 to 10 and more preferably from 1 to 5.

Examples of an alkoxy group include a methoxy group, an ethoxy group, a propyloxy group, an i-propyloxy group, an n-butoxy group, an i-butoxy group, an s-butoxy group, and a t-butoxy group.

R₂₂ in Formula (3) above is a hydrogen atom or a methyl group and preferably a hydrogen atom.

According to a preferred embodiment of the present invention, the radical-polymerizable compound comprises a polymerization unit represented by the following Formula (3-i), a polymerization unit represented by the following Formula (3-ii), or a combination thereof. Therefore, the polymerization unit represented by Formula (3) may be a polymerization unit represented by the following Formula (3-i), a polymerization unit represented by the following Formula (3-ii), or a combination thereof. Here, in Formula (3-i) and Formula (3-ii) above, R₁ to R₂₀, and R₂₂ may be different from one another.

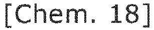

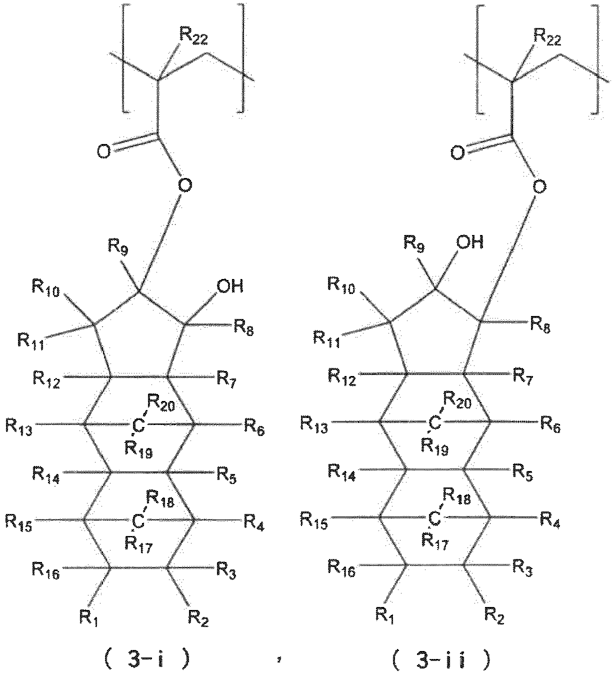

According to a preferred embodiment of the present invention, in Formula (3) above, one of R₁ and R₂ is a methacryloyloxy group and the other of R₁ and R₂, R₃ to R₂₀, and R₂₂ are each a hydrogen atom. A more preferred embodiment of the above is represented by the following Formulas (3-i-1) and (3-ii-1). Specifically, Formulas (3-i-1) and (3-ii-1) show that any one of groups corresponding to R₁ and R₂ bound to the norbornane skeleton in Formula (3) is a methacryloyloxy group.

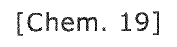

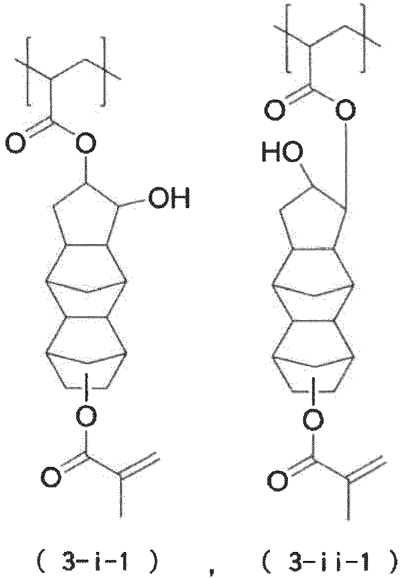

According to one embodiment of the present invention, the polymerization unit represented by Formula (3-i-1) is a polymerization unit represented by the following Formula (3-i-1-1), a polymerization unit represented by the following Formula (3-i-1-2), or a combination thereof. The polymerization unit represented by Formula (3-ii-1) is a polymerization unit represented by the following Formula (3-ii-1-1), a polymerization unit represented by the following Formula (3-ii-1-2), or a combination thereof.

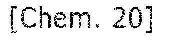

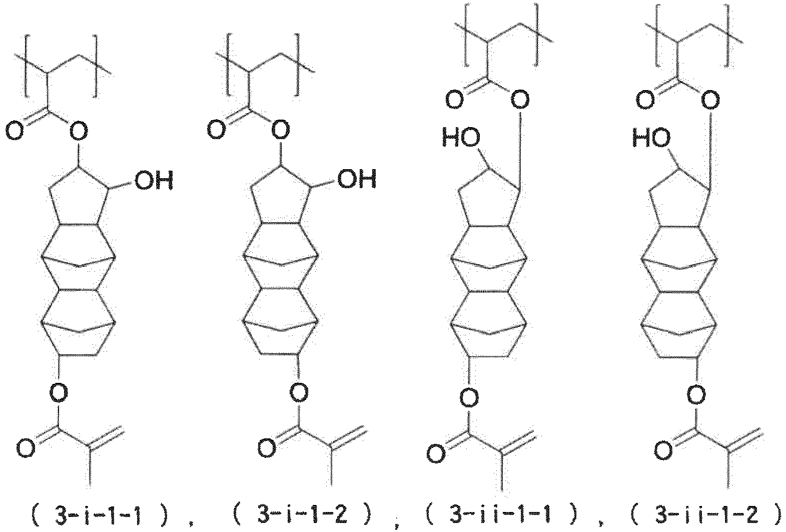

According to one embodiment of the present invention, as the polymerization unit represented by Formula (3-i-1), a combination of the polymerization unit represented by Formula (3-i-1-1) and the polymerization unit represented by (3-i-1-2) can be mentioned.

According to one embodiment of the present invention, the radical-polymerizable compound comprises the polymerization unit represented by Formula (3-i-1-1), the polymerization unit represented by Formula (3-i-1-2), or a combination thereof.

According to one embodiment of the present invention, the radical-polymerizable compound comprises the polymerization unit represented by (3-ii-1-1), the polymerization unit represented by Formula (3-ii-1-2), or a combination thereof.

According to a preferred embodiment of the present invention, the above-described polymerization unit represented by Formula (3-i-1) is the polymerization unit represented by Formula (3-i-1-2).

According to a preferred embodiment of the present invention, the above-described polymerization unit represented by Formula (3-ii-1) is the polymerization unit represented by Formula (3-ii-1-2).

Note that, according to one embodiment of the present invention, the above-described polymerization unit represented by Formula (3) may be a combination of polymerization units in which R₁ to R₂₀ and R₂₂ are different from one another. The present invention also encompasses such an aspect.

According to one embodiment of the present invention, the radical-polymerizable compound further comprises a polymerization unit derived from at least one acrylic compound selected from (meth)acrylic acid ester and (meth)acrylic acid, together with the polymerization unit represented by Formula (3). Examples of such an acrylic compound include (meth)acrylic acid alkyl esters including C₁₋₄ alkyl(meth) acrylates such as (meth)acrylic acid, methyl (meth)acrylate, ethyl (meth)acrylate, and butyl(meth)acrylate, which are (meth)acrylic acid esters, and (meth)acrylic acid. Preferred examples of the polymerization unit include a methyl methacrylate-derived polymerization unit.

Therefore, according to one embodiment of the present invention, the radical-polymerizable compound may further comprise a polymerization unit represented by the following Formula (3-1) together with the polymerization unit represented by Formula (3). Here, the above-described radical-polymerizable compound may comprise a plurality of polymerization units in which R₂₃ and R₂₄ are different. 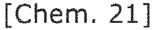 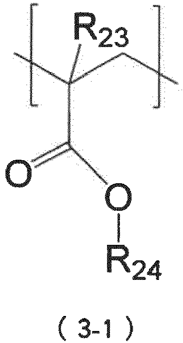 wherein, R₂₃ is selected from a hydrogen atom and a methyl group, and
R₂₄ is selected from a hydrogen atom and an alkyl group.

In the polymerization unit represented by Formula (3-1), R₂₃ is selected from a hydrogen atom or a methyl group and is preferably a methyl group.

The number of carbon atoms in an alkyl group of R₂₄ in the above-described Formula (3-1) is, but is not particularly limited to, preferably from 1 to 10 and more preferably from 1 to 5. The above-described alkyl groups may be a linear alkyl group or branched alkyl group, but is preferably a linear alkyl group.

Examples of the linear alkyl group include a methyl group, an ethyl group, a propyl group, and an n-butyl group, and preferably a methyl group.

Examples of the branched alkyl group include an isopropyl group, an isobutyl group, and a t-butyl group.

In Formula (3-1), it is more preferable that R₂₃ is a methyl group and R₂₄ is a methyl group.

According to one embodiment of the present invention, the content of the polymerization unit represented by Formula (3) in the radical-polymerizable compound can be from 10 to 90 mol%, preferably from 20 to 80 mol%, and more preferably from 30 to 70 mol% with respect to the total amount (100 mol%) of all polymerization units in the radical-polymerizable compound.

According to one embodiment of the present invention, the content of the polymerization unit represented by Formula (3-1) in the radical-polymerizable compound can be from 90 to 10 mol%, preferably from 80 to 20 mol%, and more preferably from 70 to 30 mol% with respect to the total amount (100 mol%) of all polymerization units in the radical-polymerizable compound.

According to another embodiment of the present invention, the butyl(meth)acrylate of the polymerization unit represented by Formula (3) and the polymerization unit represented by Formula (3-1) (the polymerization unit represented by Formula (3) : the polymerization unit represented by Formula (3-1)) in the radical-polymerizable compound is preferably from 1:0.05 to 1:10 and more preferably from 1:0.1 to 1:1 on a mass basis. In addition, the content ratio (the polymerization unit represented by Formula (3) : the polymerization unit represented by Formula (3-1)) is preferably from 1: 0.1 to 1:10, and more preferably from 1:0.5 to 1:1 on a mole basis.

According to one embodiment of the present invention, the number average molecular weight (Mn) of the radical-polymerizable compound is, for example, 500 or more, and it can be preferably from 1,000 to 200,000 and more preferably from 3,000 to 30, 000. The number average molecular weight can be measured by size exclusion chromatography.

According to one embodiment of the present invention, the weight average molecular weight (Mw) of the radical-polymerizable compound is, for example, 5,000 or more, and it can be preferably from 3,000 to 200,000 and more preferably from 5,000 to 50,000. The weight average molecular weight can be measured by size exclusion chromatography.

### Method of Producing Radical-polymerizable Compound

According to one embodiment of the present invention, the radical-polymerizable compound containing the polymerization unit of Formula (3) can be obtained by a method comprising a step (ii) of reacting a polymer (hereinafter, also referred to as "trunk polymer") of at least one acrylic compound selected from (meth)acrylic acid ester and (meth)acrylic acid with the above-described alicyclic epoxy acrylate compound of Formula (1-2).

According to a preferred embodiment of the present invention, the method of producing the radical-polymerizable compound containing the polymerization unit of Formula (3) may comprise a step (i) of preparing a trunk polymer before the step (ii) of reacting the above-described trunk polymer with the alicyclic epoxy acrylate compound.

### Trunk Polymer Preparation Step

The above-described trunk polymer preparation step is a step of performing radical polymerization of a polymer of at least one acrylic compound selected from (meth)acrylic acid ester and (meth)acrylic acid, and a method of radical polymerization of a normal acrylic resin can be appropriately selected according to the common technical knowledge widely known to those skilled in the art.

According to one embodiment of the present invention, the trunk polymer preparation step may be preferably a step of performing radical polymerization of at least one acrylic compound selected from (meth)acrylic acid ester and (meth)acrylic acid in the presence of a photoradical polymerization initiator or a thermal radical polymerization initiator. Specific examples of the photoradical polymerization initiator and the thermal radical polymerization initiator described above include a photoradical polymerization initiator and a thermal radical polymerization initiator which are used for a curable composition described later.

Examples of the above-described at least one acrylic compound selected from (meth)acrylic acid ester and (meth)acrylic acid include: (meth)acrylic acid alkyl esters including C₁₋₄ alkyl(meth) acrylates such as methyl (meth)acrylate, ethyl (meth)acrylate, and butyl(meth)acrylate, which are (meth)acrylic acid esters, (meth)acrylic acid, or combinations thereof, and preferably methyl (meth)acrylate, (meth)acrylic acid, or a combination thereof, and more preferably a combination of methyl methacrylate and acrylic acid.

Therefore, examples of a polymer (trunk polymer) of at least one acrylic compound selected from (meth)acrylic acid ester and (meth)acrylic acid which is obtained in the trunk polymer preparation step include a (co)polymer of (meth)acrylic acid ester, a (co)polymer of (meth)acrylic acid, and a (co)polymer of (meth)acrylic acid ester and (meth)acrylic acid, and preferably a copolymer of (meth)acrylic acid ester and (meth)acrylic acid and more preferably a copolymer of methacrylic acid ester and acrylic acid. Specific examples of such trunk polymers include (meth)acrylic acid alkyl esters including C₁₋₄ alkyl(meth) acrylates such as methyl (meth)acrylate, ethyl (meth)acrylate, and butyl(meth)acrylate, or polymers of combinations thereof, and preferably a copolymer of methyl (meth)acrylate and (meth)acrylic acid and more preferably a copolymer of methyl methacrylate and acrylic acid.

According to one embodiment of the present invention, the amount of a photoradical polymerization initiator or a thermal radical polymerization initiator used in the trunk polymer preparation step is preferably from 0.1 to 20 mol% and more preferably from 1 to 10 mol% with respect to the acrylic compound.

In the case of using a thermal radical polymerization initiator as the radical polymerization initiator, it is preferable to perform agitation in the presence of a solvent at 50°C to 150°C for 1 to 10 hours, for example, in the above-described radical polymerization step.

In the case of using a photoradical polymerization initiator as the radical polymerization initiator, the cumulative irradiation dose (accumulated amount of light) of an active energy ray may be, for example, from 100 to 5000 mJ/cm² and is preferably from 300 to 4000 mJ/cm² in the above-described radical polymerization step. Here, the cumulative irradiation dose (accumulated amount of light) refers to the irradiation dose expressed by the product of irradiation intensity and irradiation time of an active energy ray (e.g., visible ray, UV ray, X-ray, or electron beam)

### Radical-polymerizable Compound Preparation Step

According to one embodiment of the present invention, the method of producing the radical-polymerizable compound containing the polymerization unit of Formula (3) comprises the step (ii) of reacting a trunk polymer with the above-described alicyclic epoxy acrylate compound of Formula (1-2).

According to one embodiment of the present invention, in the production of the radical-polymerizable compound containing the polymerization unit of Formula (3), it is preferable to perform purification by distillation, a column, or recrystallization in cases where the purity of a radical-polymerizable compound obtained by the above-described method is low.

According to one embodiment of the present invention, the amount of the alicyclic epoxy acrylate compound of Formula (1-2) used in the production of the radical-polymerizable compound is, but is not particularly limited to, preferably from 0.50 to 2.00 mol and more preferably from 0.80 to 1.50 mol with respect to 1.00 mol of carboxylic acid in the trunk polymer.

According to one embodiment of the present invention, it is preferable to perform agitation at 80°C to 150°C for 4 to 10 hours, for example, in the above-described radical polymerization step.

According to one embodiment of the present invention, it is preferable to produce the radical-polymerizable compound in the presence of a solvent (e.g., Pph3 or PBu3) and/or a polymerization inhibitor (e.g., hydroquinone monomethylether, hydroquinone, or 4-methoxyphenol) as appropriate.

The amount of the catalyst used is usually from 0.01 to 10 mol%, preferably from 0.1 to 5 mol%, based on 1 mol of the alicyclic epoxy acrylate compound of Formula (1-2).

The amount of the polymerization inhibitor used is usually from 0.01 to 10 mol%, preferably from 0.1 to 5 mol%, based on 1 mol of the alicyclic epoxy acrylate compound of Formula (1-2).

### Usefulness of Radical-polymerizable Compound

According to one embodiment of the present invention, when allowing the curable composition to contain the radical-polymerizable compound, a cured product of the curable composition can have high heat resistance. Therefore, it can be used as with the above-described alicyclic epoxy acrylate compound.

### Curable Composition

According to the present invention, a curable composition having high heat resistance can be provided by combining an alicyclic acrylate-type polymerizable monomer comprising the above-described alicyclic epoxy acrylate compound represented by Formula (1-2) and an epoxy group-containing (meth)acrylic polymer containing the polymerization unit of Formula (2) or a radical-polymerizable compound containing the polymerization unit of Formula (3) optionally together with other components (e.g., a curing agent, a thermal cationic polymerization initiator, a photo-cationic polymerization initiator, a thermal radical polymerization initiator, an epoxy compound which is different from the above-described alicyclic epoxy acrylate compound and/or epoxy group-containing (meth)acrylic polymer, and a radical-polymerizable compound which is different from the above-described alicyclic epoxy acrylate compound and/or a radical-polymerizable compound containing the polymerization unit of Formula (3)). Hereinafter, the curable composition will be specifically described.

### Curable Composition Comprising Alicyclic Acrylate-type Polymerizable Monomer Containing Alicyclic Epoxy Acrylate Compound Represented by Formula (1-2)

According to one embodiment of the present invention, the curable composition is characterized in that it comprises at least an alicyclic acrylate-type polymerizable monomer containing the above-described alicyclic epoxy acrylate compound represented by Formula (1-2).

In addition, it is preferable that the above-described curable composition further comprises at least one selected from the group consisting of a curing agent, a thermal cationic polymerization initiator, and a photo-cationic polymerization initiator, and more preferably further comprises at least one selected from the group consisting of a curing agent, a thermal cationic polymerization initiator, and a photo-cationic polymerization initiator.

Further, it is preferable that the above-described curable composition further comprises a thermal radical polymerization initiator.

Still further, it is preferable that the above-described curable composition further comprises an epoxy compound which is different from the above-described alicyclic epoxy acrylate compound.

Yet further, it is preferable that the above-described curable composition further comprises a radical-polymerizable compound which is different from the above-described alicyclic epoxy acrylate compound.

According to one embodiment of the present invention, the proportion of the alicyclic acrylate-type polymerizable monomer comprising the alicyclic epoxy acrylate compound of Formula (1-2) blended in the curable composition is such that the alicyclic epoxy acrylate compound of Formula (1-2) is within the range of from 10 to 80 parts by mass and more preferably from 15 to 65 parts by mass with respect to 100 parts by mass in total of the curable composition. A cured product that is further excellent in heat resistance can be obtained by allowing the alicyclic acrylate-type polymerizable monomer comprising the alicyclic epoxy acrylate compound of Formula (1-2) to be contained in this range.

According to another preferred embodiment of the present invention, the curable composition comprises the alicyclic acrylate-type polymerizable monomer comprising the above-described alicyclic epoxy acrylate compound represented by Formula (1-2) and at least one selected from a curing agent and a thermal cationic polymerization initiator. By allowing the curable composition to comprise the alicyclic acrylate-type polymerizable monomer containing the above-described alicyclic epoxy acrylate compound represented by Formula (1-2), heat resistance of a cured product formed by curing the curable composition can be further improved. Further, it is advantageous in that transparency of a cured product formed by curing the above-described curable composition can be improved.

### Curable Composition Comprising Alicyclic Acrylate-type Polymerizable Monomer Containing Alicyclic Epoxy Acrylate Compound Represented by Formula (1-2) (Curable Composition 1)

According to another preferred embodiment of the present invention, the curable composition comprises an alicyclic acrylate-type polymerizable monomer comprising the alicyclic epoxy acrylate compound of Formula (1-2), an epoxy compound that is different from the alicyclic epoxy acrylate compound of Formula (1-2), a radical-polymerizable compound that is different from the alicyclic epoxy acrylate compound of Formula (1-2), a thermal cationic polymerization initiator, and a thermal radical polymerization initiator (hereinafter, also referred to as "curable composition 1"). A cured product formed by curing the above-described curable composition can have high heat resistance. Further, it is advantageous in that transparency of a cured product formed by curing the above-described curable composition can be improved.

### Curable Composition Comprising Alicyclic Acrylate-type Polymerizable Monomer Containing Alicyclic Epoxy Acrylate Compound Represented by Formula (1-2) (Curable Composition 2)

According to another preferred embodiment of the present invention, the curable composition comprises an alicyclic acrylate-type polymerizable monomer comprising the alicyclic epoxy acrylate compound of Formula (1-2), a radical-polymerizable compound that is different from the alicyclic epoxy acrylate compound of Formula (1-2), a curing agent, and a thermal radical polymerization initiator (hereinafter, also referred to as "curable composition 2"). The above-described curable composition may further contain a curing accelerator. A cured product formed by curing the above-described curable composition can have high heat resistance. Further, it is advantageous in that transparency of a cured product formed by curing the above-described curable composition can be improved.

### Curable Composition Comprising Alicyclic Acrylate-type Polymerizable Monomer Containing Alicyclic Epoxy Acrylate Compound Represented by Formula (1-2) (Curable Composition 3)

According to another preferred embodiment of the present invention, the curable composition comprises an alicyclic acrylate-type polymerizable monomer comprising the alicyclic epoxy acrylate compound of Formula (1-2), an epoxy compound that is different from the alicyclic epoxy acrylate compound of Formula (1-2), and a photo-cationic polymerization initiator (hereinafter, also referred to as "curable composition 3"). The above-described curable composition may further contain a photosensitizer. A cured product formed by curing the above-described curable composition can have high heat resistance.

### Curable Composition Comprising Epoxy Group-Containing (Meth)acrylic Polymer Containing Polymerization Unit of Formula (2)

According to one embodiment of the present invention, the curable composition is characterized in that it comprises at least an epoxy group-containing (meth)acrylic polymer containing the above-described polymerization unit of Formula (2).

Further, it is preferable that the above-described curable composition further comprises at least one selected from the group consisting of a curing agent, a thermal cationic polymerization initiator, and a photo-cationic polymerization initiator.

Still further, it is preferable that the above-described curable composition further comprises an epoxy compound that is different from the above-described epoxy group-containing (meth)acrylic polymer.

According to one embodiment of the present invention, the proportion of the epoxy group-containing (meth)acrylic polymer containing the polymerization unit of Formula (2) blended in the curable composition of the present invention is such that the epoxy group-containing (meth)acrylic polymer is within the range of preferably from 5 to 80 parts by mass and more preferably from 15 to 65 parts by mass with respect to 100 parts by mass in total of the curable composition. A cured product that is further excellent in heat resistance can be obtained by allowing the epoxy group-containing (meth)acrylic polymer containing the polymerization unit of Formula (2) to be contained in this range.

### Curable Composition Comprising Epoxy Group-Containing (Meth)acrylic Polymer Containing Polymerization Unit of Formula (2) (Curable Composition 4)

According to a preferred embodiment of the present invention, the curable composition comprises an epoxy group-containing (meth)acrylic polymer containing the polymerization unit of Formula (2) and a photo-cationic polymerization initiator (hereinafter, also referred to as "curable composition 4"). A cured product formed by curing the above-described curable composition can have high heat resistance.

### Curable Composition Comprising Radical-polymerizable Compound Containing Polymerization Unit of Formula (3)

According to one embodiment of the present invention, the curable composition is characterized in that it comprises a radical-polymerizable compound containing at least the polymerization unit of Formula (3).

In addition, it is preferable that the above-described curable composition further comprises at least one selected from the group consisting of a curing agent, a thermal cationic polymerization initiator, and a photo-cationic polymerization initiator.

Further, it is preferable that the above-described curable composition comprises a thermal radical polymerization initiator or a photoradical polymerization initiator.

Still further, it is preferable that the above-described curable composition further comprises a radical-polymerizable compound that is different from the radical-polymerizable compound containing the above-described polymerization unit of Formula (3).

According to one embodiment of the present invention, the proportion of the radical-polymerizable compound containing the polymerization unit of Formula (3) blended in the curable composition is such that the radical-polymerizable compound is within the range of from 10 to 90 parts by mass and more preferably from 30 to 80 parts by mass with respect to 100 parts by mass of the curable composition. A cured product that is further excellent in heat resistance can be obtained by allowing the radical-polymerizable compound containing the polymerization unit of Formula (3) to be contained in this range.

### Curable Composition Comprising Radical-polymerizable Compound Containing Polymerization Unit of Formula (3) (Curable Composition 5)

According to a preferred embodiment of the present invention, the curable composition comprises the radical-polymerizable compound comprising the polymerization unit of Formula (3), a radical-polymerizable compound that is different from the above-described radical-polymerizable compound, and a photoradical polymerization initiator (I) (hereinafter, also referred to as "curable composition 5"). The above-described curable composition may further contain a photosensitizer. A cured product formed by curing the above-described curable composition can have high heat resistance.

### Components which Can Be Contained in Curable Composition Curing Agent

According to one embodiment of the present invention, examples of the curing agent which can be contained in the curable composition include an acid anhydride-based compound, an amine-based compound, a phenol-based compound, and a latent curing agent.

Examples of the acid anhydride-based compound include hexahydrophthalic anhydride, methylhexahydrophthalic anhydride, tetrahydrophthalic anhydride, methyltetrahydrophthalic anhydride, endomethylenetetrahydrophthalic anhydride, methylnadic anhydride, methylbutenyltetrahydrophthalic anhydride, hydrogenated methylnadic anhydride, trialkyltetrahydrophthalic anhydride, cyclohexanetricarboxylic anhydride, methylcyclohexenedicarboxylic anhydride, methylcyclohexanetetracarboxylic acid dianhydride, maleic anhydride, phthalic anhydride, succinic anhydride, dodecenylsuccinic anhydride, octenylsuccinic anhydride, pyromellitic anhydride, trimellitic anhydride, alkylstyrene-maleic anhydride copolymer, chlorendic anhydride, polyazelaic anhydride, benzophenone tetracarboxylic anhydride, ethylene glycol bisanhydrotrimellitate, glycerol tristrimellitate, glycerin bis(anhydrotrimellitate) monoacetate, benzophenonetetracarboxylic acid, polyadipic anhydride, polysebacic anhydride, poly(ethyloctadecanedioic acid) anhydride, poly(phenylhexadecanedioic acid) anhydride, and norbornane-2,3-dicarboxylic anhydride, and preferably hexahydrophthalic anhydride, methylhexahydrophthalic anhydride, or a combination thereof.

Examples of the amine-based compound include polyoxyethylene diamine, polyoxypropylene diamine, polyoxybutylene diamine, polyoxypentylene diamine, polyoxyethylene triamine, polyoxypropylene triamine, polyoxybutylene triamine, polyoxypentylene triamine, diethylene triamine, triethylene tetramine, tetraethylene pentamine, m-xylene diamine, trimethylhexamethylene diamine, 2-methylpentamethylene diamine, diethylaminopropylamine, isophorone diamine, 1,3-bisaminomethylcyclohexane, bis(4-aminocyclohexyl)methane, norbornane diamine, 1,2-diaminocyclohexane, diaminodiphenylmethane, metaphenylene diamine, diaminodiphenyl sulfone, and N-aminoethylpiperazine.

Examples of the phenol-based compound include xylylene skeleton-containing phenol novolac resins, dicyclopentadiene skeleton-containing phenol novolac resins, biphenyl skeleton-containing phenol novolac resins, fluorene skeleton-containing phenol novolac resins, terpene skeleton-containing phenol novolac resins, bisphenol A novolac, bisphenol F novolac, bisphenol S novolac, bisphenol AP novolac, bisphenol C novolac, bisphenol E novolac, bisphenol Z novolac, biphenol novolac, tetramethyl bisphenol A novolac, dimethyl bisphenol A novolac, tetramethyl bisphenol F novolac, dimethyl bisphenol F novolac, tetramethyl bisphenol S novolac, dimethyl bisphenol S novolac, tetramethyl-4,4'-biphenol novolac, trishydroxyphenylmethane novolac, resorcinol novolac, hydroquinone novolac, pyrogallol novolac, diisopropylidene novolac, 1,1-di-4-hydroxyphenylfluorene novolac, phenolated polybutadiene novolac, phenol novolac, cresol novolac, ethylphenol novolac, butylphenol novolac, octylphenol novolac, and naphthol novolac.

Examples of the latent curing agent include dicyandiamide, adipic acid dihydrazide, sebacic acid dihydrazide, dodecanedioic acid dihydrazide, isophthalic acid dihydrazide, ketimines, imidazole compounds, dihydrazide compounds, amine adduct-based latent curing agents. The curable composition may contain one kind, or two or more kinds of the curing agents as described above.

According to a preferred embodiment of the curable composition of the present invention, the curing agent is one or more curing agents selected from the group consisting of acid anhydride-based compounds, amine-based compounds, phenol-based compounds, and latent curing agents, and it is more preferably an acid anhydride-based compound.

According to one embodiment of the present invention, the proportion of the curing agent blended in the curable composition is such that the curing agent is within the range of from 10 to 80 parts by mass and more preferably from 20 to 60 parts by mass with respect to 100 parts by mass in total of the curable composition. A cured product that is further excellent in heat resistance can be obtained by allowing the curing agent to be contained in this range.

### Thermal Cationic Polymerization Initiator

According to one embodiment of the present invention, examples of the thermal cationic polymerization initiator which can be contained in the curable composition include thermal cationic polymerization initiators such as: an onium salt composed of at least one cation selected from aromatic sulfonium, aromatic iodonium, aromatic diazonium, pyridinium, and the like and at least one anion selected from BF₄⁻, PF₆⁻, SbF₆⁻, AsF₆⁻, CF₃SO₃⁻, (CF₃SO₂)₂N⁻, and B(C₆F₅)₄⁻; and an aluminum complex.

Examples of the aromatic sulfonium salt-based thermal cationic polymerization initiator include: hexafluoroantimonate salts such as (2-ethoxy-1-methyl-2-oxoethyl)methyl-2-naphthalenylsulfonium hexafluoroantimonate, 4-(methoxycarbonyloxy)phenylbenzylmethylsulfonium hexafluoroantimonate, 4-acetoxyphenyldimethylsulfonium hexafluoroantimonate, 4-hydroxyphenylbenzylmethylsulfonium hexafluoroantimonate, 4-hydroxyphenyl(o-methylbenzyl)methylsulfonium hexafluoroantimonate, 4-hydroxyphenyl(a-naphthylmethyl)methylsulfonium hexafluoroantimonate, diphenyl-4-(phenylthio)phenylsulfonium hexafluoroantimonate, triphenylsulfonium hexafluoroantimonate, bis[4-(di(4-(2-hydroxyethoxy))phenylsulfonio)phenyl]sulfide bishexafluoroantimonate, and bis[4-(diphenylsulfonio)phenyl]sulfide bishexafluoroantimonate; hexafluorophosphate salts such as (2-ethoxy-1-methyl-2-oxoethyl)methyl-2-naphthalenylsulfonium hexafluorophosphate, 4-acetoxyphenylbenzylmethylsulfonium hexafluorophosphate, 4-hydroxyphenyl(o-methylbenzyl)methylsulfonium hexafluorophosphate, 4-hydroxyphenyl(a-naphthylmethyl)methylsulfonium hexafluorophosphate, diphenyl-4-(phenylthio)phenylsulfonium hexafluorophosphate, triphenylsulfonium hexafluorophosphate, bis[4-(di(4-(2-hydroxyethoxy))phenylsulfonio)phenyl]sulfide bishexafluorophosphate, and bis[4-(diphenylsulfonio)phenyl]sulfide bishexafluorophosphate; hexafluoroarsenate salts such as 4-hydroxyphenyl(o-methylbenzyl)methylsulfonium hexafluoroarsenate, and 4-hydroxyphenylbenzylmethylsulfonium hexafluoroarsenate; tetrafluoroborate salts such as (2-ethoxy-1-methyl-2-oxoethyl)methyl-2-naphthalenylsulfonium tetrafluoroborate, 4-hydroxyphenyl(o-methylbenzyl)methylsulfonium tetrafluoroborate, 4-hydroxyphenylbenzylmethylsulfonium tetrafluoroborate, diphenyl-4-(phenylthio)phenylsulfonium tetrafluoroborate, triphenylsulfonium tetrafluoroborate, bis[4-(di(4-(2-hydroxyethoxy))phenylsulfonio)phenyl]sulfide bistetrafluoroborate, and bis[4-(diphenylsulfonio)phenyl]sulfide bistetrafluoroborate; trifluoromethanesulfonate salts such as 4-hydroxyphenyl(o-methylbenzyl)methylsulfonium trifluoromethanesulfonate, and 4-hydroxyphenylbenzylmethylsulfonium trifluoromethanesulfonate; trifluoromethanesulfonate salts such as diphenyl-4-(phenylthio)phenylsulfonium trifluoromethanesulfonate; bis(trifluoromethanesulfone)imide salts such as 4-hydroxyphenyl(a-naphthylmethyl)methylsulfonium bis(trifluoromethanesulfone)imide, and 4-hydroxyphenylbenzylmethylsulfonium bis(trifluoromethanesulfone)imide; tetrakis(pentafluorophenyl)borate salts such as (2-ethoxy-1-methyl-2-oxoethyl)methyl-2-naphthalenylsulfonium tetrakis(pentafluorophenyl)borate, 4-(methoxycarbonyloxy)phenylbenzylmethylsulfonium tetrakis(pentafluorophenyl) borate, 4-hydroxyphenyl(o-methylbenzyl)methylsulfonium tetrakis(pentafluorophenyl)borate, 4-hydroxyphenyl(a-naphthylmethyl)methylsulfonium tetrakis(pentafluorophenyl)borate, 4-hydroxyphenylbenzylmethylsulfonium tetrakis(pentafluorophenyl)borate, diphenyl-4-(phenylthio)phenylsulfonium tetrakis(pentafluorophenyl)borate, triphenylsulfonium tetrakis(pentafluorophenyl)borate, bis[4-(di(4-(2-hydroxyethoxy))phenylsulfonio)phenyl]sulfide tetrakis(pentafluorophenyl)borate, and bis[4-(diphenylsulfonio)phenyl]sulfide tetrakis(pentafluorophenyl)borate. Monoaryl-based thermal cationic polymerization initiators such as 4-acetoxyphenyldimethylsulfonium hexafluoroantimonate are preferred.

Examples of the aromatic iodonium salt-based thermal cationic polymerization initiator include phenyliodonium hexafluorophosphate, diphenyliodonium hexafluoroantimonate, diphenyliodonium tetrafluoroborate, diphenyliodonium tetrakis(pentafluorophenyl)borate, diphenyliodonium hexafluorophosphate, diphenyliodonium trifluoromethanesulfonate, bis(dodecylphenyl)iodonium hexafluorophosphate, bis(dodecylphenyl)iodonium hexafluoroantimonate, bis(dodecylphenyl)iodonium tetrafluoroborate, bis(dodecylphenyl)iodonium tetrakis(pentafluorophenyl)borate, 4-methylphenyl-4-(1-methylethyl)phenyliodonium hexafluorophosphate, 4-methylphenyl-4-(1-methylethyl)phenyliodonium hexafluoroantimonate, 4-methylphenyl-4-(1-methylethyl)phenyliodonium tetrafluoroborate, and 4-methylphenyl-4-(1-methylethyl)phenyliodonium tetrakis(pentafluorophenyl)borate.

Examples of the aromatic diazonium salt-based thermal cationic polymerization initiator include phenyldiazonium hexafluorophosphate, phenyldiazonium hexafluoroantimonate, phenyldiazonium tetrafluoroborate and phenyldiazonium tetrakis(pentafluorophenyl)borate.

Examples of the pyridinium salt-based thermal cationic polymerization initiator include 1-benzyl-2-cyanopyridinium hexafluorophosphate, 1-benzyl-2-cyanopyridinium hexafluoroantimonate, 1-benzyl-2-cyanopyridinium tetrafluoroborate, 1-benzyl-2-cyanopyridinium tetrakis(pentafluorophenyl)borate, 1-(naphthylmethyl)-2-cyanopyridinium hexafluorophosphate, 1-(naphthylmethyl)-2-cyanopyridinium hexafluoroantimonate, 1-(naphthylmethyl)-2-cyanopyridinium tetrafluoroborate, and 1-(naphthylmethyl)-2-cyanopyridinium tetrakis(pentafluorophenyl)borate.

Examples of the aluminum complex-based thermal cationic polymerization initiator include aluminum carboxylates; aluminum alkoxide, aluminium chloride, aluminum (alkoxide) acetoacetic acid chelate, acetoacetonato aluminum, and ethylacetoacetato aluminum.

Examples of the phosphonium salt-based thermal cationic polymerization initiator include ethyltriphenylphosphonium hexafluoroantimonate, and tetrabutylphosphonium hexafluoroantimonate.

Examples of the quaternary ammonium salt-based thermal cationic polymerization initiator include N,N-dimethyl-N-benzylanilinium hexafluoroantimonate, N,N-diethyl-N-benzylanilinium tetrafluoroborate, N,N-dimethyl-N-benzylpyridinium hexafluoroantimonate, N,N-diethyl-N-benzylpyridinium trifluoromethanesulfonic acid, N,N-dimethyl-N-(4-methoxybenzyl)pyridinium hexafluoroantimonate, N,N-diethyl-N-(4-methoxybenzyl)pyridinium hexafluoroantimonate, N,N-diethyl-N-(4-methoxybenzyl)toluidinium hexafluoroantimonate, and N,N-dimethyl-N-(4-methoxybenzyl)toluidinium hexafluoroantimonate.

According to one embodiment of the present invention, the curable composition may contain one kind, or two or more kinds of thermal cationic polymerization initiators as described above.

According to a preferred embodiment of the curable composition of the present invention, the thermal cationic polymerization initiator is preferably selected from the group consisting of aromatic sulfonium salt-based thermal cationic polymerization initiators, aromatic iodonium salt-based thermal cationic polymerization initiators and aluminum complex-based thermal cationic polymerization initiators, and more preferably is an aromatic sulfonium salt-based thermal cationic polymerization initiator.

According to one embodiment of the present invention, the proportion of the thermal cationic polymerization initiator blended in the curable composition is such that the thermal cationic polymerization initiator is within the range of from 0.1 to 20 parts by mass and more preferably from 0.5 to 10 parts by mass with respect to 100 parts by mass in total of the curable composition. A cured product that is further excellent in heat resistance can be obtained by allowing the thermal cationic polymerization initiator to be contained in this range.

### Photo-cationic Polymerization Initiator

According to one embodiment of the present invention, the photo-cationic polymerization initiator that may be contained in the curable composition is one which generates cationic species or Lewis acid when irradiated with an active energy ray such as a visible ray, UV ray, X-ray, or electron beam, thereby initiating a polymerization reaction of a cationically polymerizable compound. As the photo-cationic polymerization initiator to be contained in the curable composition, it is possible to use, for example, a compound such as an onium salt, a metallocene complex, or an iron-allene complex. Examples of the onium salt which can be used include aromatic sulfonium salts, aromatic diazonium salts, aromatic phosphonium salts and aromatic selenium salts. As the counter ions for these salts, anions such as CF₃SO₃-, BF₄-, PF₆-, AsF₆-, and SbF₆- are used. Among these, it is preferred to use an aromatic sulfonium salt-based photo-cationic polymerization initiator, since it exhibits an excellent curing performance due to having UV absorption properties even in the wavelength range of 300 nm or more, and allows for providing a cured product having a good mechanical strength and adhesion strength. The curable composition may contain two or more kinds of the photo-cationic polymerization initiators.

Examples of the aromatic sulfonium salt include a diphenyl-4-(phenylthio)phenylsulfonium salt (e.g., diphenyl-4-(phenylthio)phenylsulfonium hexafluorophosphate), 4,4'-bis(diphenylsulfonio)diphenylsulfide bishexafluorophosphate, 4,4'-bis[di(β-hydroxyethoxy)phenylsulfonio]diphenylsulfide bishexafluoroantimonate, 4,4'-bis[di(β-hydroxyethoxy)phenylsulfonio]diphenylsulfide bishexafluorophosphate, 7-[di(p-toluyl)sulfonio]-2-isopropylthioxanthone hexafluoroantimonate, 7-[di(p-toluyl)sulfonio]-2-isopropylthioxanthone tetrakis(pentafluorophenyl)borate, 4-phenylcarbonyl-4'-diphenylsulfonio-diphenylsulfide hexafluorophosphate, 4-(p-tert-butylphenylcarbonyl)-4'-diphenylsulfonio-diphenylsulfi de hexafluoroantimonate, 4-(p-tert-butylphenylcarbonyl)-4'-di(p-toluyl)sulfonio-diphenyls ulfide tetrakis(pentafluorophenyl)borate, diphenyl-4-(phenylthio)phenylsulfonium hexafluoroantimonate, triphenylsulfonium trifluoromethanesulfonate, bis[4-(diphenylsulfonio)phenyl]sulfide bishexafluoroantimonate, and (4-methoxyphenyl)diphenylsulfonium hexafluoroantimonate. A diphenyl-4-(phenylthio)phenylsulfonium salt is preferred.

Examples of the aromatic diazonium salt include benzenediazonium hexafluoroantimonate, benzenediazonium hexafluorophosphate, benzenediazonium tetrafluoroborate, and 4-chlorobenzenediazonium hexafluorophosphate.

Examples of the aromatic phosphonium salt include benzyltriphenylphosphonium hexafluoroantimonate.

Examples of the aromatic selenium salt include triphenylselenium hexafluorophosphate.

Examples of the iron-allene complex include xylene-cyclopentadienyl iron (II) hexafluoroantimonate, cumene-cyclopentadienyl iron (II) hexafluorophosphate, and xylene -cyclopentadienyl iron (II) tris(trifluoromethylsulfonyl)methanide.

According to one embodiment of the present invention, the proportion of the photo-cationic polymerization initiator blended in the curable composition is such that the photo-cationic polymerization initiator is within the range of from 0.1 to 20 parts by mass and more preferably from 0.5 to 10 parts by mass with respect to 100 parts by mass in total of the curable composition. A cured product that is further excellent in heat resistance can be obtained by allowing the photo-cationic polymerization initiator to be contained in this range.

### Thermal Radical Polymerization Initiator

According to one embodiment of the present invention, the thermal radical polymerization initiator which can be contained in the curable composition is not particularly limited the present invention unless it interferes with the gist of the present invention, and thermal radical polymerization initiators such as peroxides and azo-based compounds are preferred. In addition, the above-described curable composition may contain two or more kinds of thermal radical polymerization initiators.

Examples of the peroxide include dialkyl peroxides such as α, α ' -bis(t-butylperoxy)diisopropylbenzene, dicumyl peroxide, and di-t-butylperoxide; ketone peroxides such as methyl ethyl ketone peroxide, cyclohexanone peroxide, and the like; 1,1-bis(t-butylperoxy)cyclohexane, 1,1-bis(t-butylperoxy)-2-methylcyclohexane,1,1-bis (t-butylperoxy)-3,5-trimethylcyclohexane, 1,1-bis(t-hexylperoxy)-3,5-trimethylcyclohexane and the like; hydroperoxides such as p-menthanohydroperoxide; octanoyl peroxide, lauroyl peroxide, stearyl peroxide, Diacylperoxide such as benzoyl peroxide; bis(4-t-butylcyclohexyl)peroxydicarbonate, di-2-ethoxyethylperoxydicarbonate, di-2-ethylhexylperoxydicarbonate, di-3-methoxybutylperoxycarbonate; t-butylperoxypivalate, 1,1,3,3-tetramethylbutylperoxy-2-ethylhexanoate, 2,5-dimethyl-2,5-bis (2-ethylhexanoylperoxy)hexanoic,t-hexylperoxy-2-ethylhexanoa te, t-butylperoxy-2-ethylhexanoate, t-butylperoxyisobutyrate, t-butylperoxy-3,5-trimethylhexanoate, t-butylperoxylaurate, Examples thereof include peroxy esters such as t-butylperoxyisopropylmonocarbonate, t-butylperoxy-2-ethylhexylmonocarbonate, t-butylperoxybenzoate, t-hexylperoxybenzoate, 2,5-dimethyl-2,5-bis (benzoylperoxy)hexane, and t-butylperoxyacetate, and are preferably t-butylperoxy-2-ethylhexanoate and t-butylperoxypivalate.

Examples of the azo-based compound include 2,2'-azobisisobutyronitrile, 2,2'-azobis(2, 4-dimethylvaleronitrile), 2,2'-azobis(4-methoxy-2'-dimethylvaleronitrile), and the like, and preferably 2,2'-azobisisobutyronitrile.

According to one embodiment of the present invention, the proportion of the thermal radical polymerization initiator blended in the curable composition is such that the thermal radical polymerization initiator is within the range of from 0.01 to 60 parts by mass and more preferably from 0.1 to 40 parts by mass with respect to 100 parts by mass of the curable composition. A cured product that is further excellent in heat resistance can be obtained by allowing the thermal radical polymerization initiator to be contained in this range.

### Photoradical Polymerization Initiator

According to one embodiment of the present invention, the photoradical polymerization initiator which can be contained in the curable composition is not particularly limited unless it interferes with the gist of the present invention. Examples thereof include photoradical polymerization initiators such as alkylphenones, anthraquinones, thioxanthones, ketals, phosphinoxides, titanocenes, imidazoles, triazines, oximes, and alkylphenones are preferred. In addition, the above-described curable composition may contain two or more kinds of photoradical polymerization initiators.

As alkylphenones, for example, benzophenone, acetophenone benzyl dimethylketone, benzoin, benzoin methyl ether, benzoin ethyl ether, dimethoxyacetophenone, diethoxyacetophenone, tetra(t-butylperoxycarbonyl)benzophenone, benzyl, 2,2-dimethoxy-1,2-diphenylethan-1-one, 1-hydroxy-cyclohexyl-phenyl-ketone,2-hydroxy-2-methyl-1-phe nyl-propan-1-one, 1-[4-(2-hydroxyethoxy)-phenyl]-2-hydroxy-2-methyl-1-propanone, 2-hyrodoxy-1-{4-[4-(2-hydroxy-2-methyl-propionyl)-benzyl]phe nyl}-2-methyl-propan-1-one, phenylglyoxylic acid methyl ester, 2-methyl-1-[4-(methyl)phenyl]-2-morpholinopropanone-1, 2-methyl-1-(4-methylthiophenyl)-2-morpholinopropan-1-one, 2-dimethylamino-2-(4-morpholino)benzoyl-1-phenylpropane, 2-benzyl-2-dimethylamino-1-(4-morpholinophenyl)-butanone-1, 2-(dimethylamino)-2-[(4-methylphenyl)methyl]-1-(4-morpholin yl)phenyl]-1-butanonebenzoin, benzoin ethyl ether, benzoin isopropyl ether, acetophenone, 2,2-dimethoxy-2-phenylacetophenone, 1,1-dichloroacetophenone, 2-methyl-1-[4-(methylthio)phenyl]-2-morpholinopropan-1-one, 2-benzyl-2-dimethylamino-1-(4-morpholinophenyl)-butanone-1, 2-(dimethylamino)-2-[(4-methylphenyl)methyl]-1-[4-(4-morpho linyl)phenyl]-1-butanone, 2-methylbenzophenone, 4,6-trimethylbenzophenone, 4-(methylphenylthio)phenylphenylmethane, methyl-2-benzophenone,1-[4-(4-benzoylphenyl)phenyl]-2-meth yl-2-(4-methylphenylsulfonyl)propan-1-one, 4,4'-bis(dimethylamino)benzophenone, N,N'-tetramethyl-4,4'-diaminobenzophenone, N, Examples thereof include N'-tetraethyl-4,4'-diaminobenzophenone and 4-methoxy-4'-dimethylaminobenzophenone, and the like, and are preferably 2-benzyl-2-dimethylamino-1-(4-morpholinophenyl)-butanone-1.

Examples of the anthraquinones include 2-methylanthraquinone, 2-ethylanthraquinone, 2-tertiary butylanthraquinone, and 1-chloroanthraquinone.

Examples of the thioxanthone include 2,4-dimethylthioxanthone, 2,4-diethylthioxanthone, 2-chlorothioxanthone, 2,4-diisopropylthioxanthone, thioxanthone, isopropylthioxanthone, 1-chloro-4-propylthioxanthone, 3-[3,4-dimethyl-9-oxo-9H-thioxanthone-2-yl]oxy]-2-hydroxypro pyl-N,N,N-trimethylammonium chloride, and phlorothioxanthone.

Examples of the ketal include acetophenone dimethyl ketal and benzyl dimethyl ketal.

Examples of the phosphine oxides include (2,6-dimethoxybenzoyl)-2,4,4-pentylphosphine oxide, bis(2,4,6-trimethylbenzoyl)-phenylphosphine oxide, 2,4,6-trimethylbenzoyldiphenylphosphine oxide, and ethyl-2,4,6-trimethylbenzoylphenylphosphinate.

Examples of the titanocenes include bis(η5-2,4-cyclopentadiene-1-yl)-bis[2,6-difluoro-3-(1H-pyrrole -1-yl)-phenyl]titanium and the like.

As imidazoles, for example, 2-(o-chlorophenyl)-4,5-diphenylimidazole, 2-(o-chlorophenyl)-4,5-di(m-methoxyphenyl)imidazole, 2-(o-methoxyphenyl)-4,5-diphenylimidazole,2-(p-methoxyphen yl)-4,5-diphenylimidazole,Examples thereof include 2,4-di(p-methoxyphenyl)-5-phenylimidazole and 2-(2,4-dimethoxyphenyl)-4,5-diphenylimidazole.

Examples of the triazines include 2,4-trichloromethyl-(4"-methoxyphenyl)-6-triazine, 2,4-trichloromethyl-(4'-methoxynaphthyl)-6-triazine, 2,4-trichloromethyl-(piperonyl)-6-triazine, and 2,4-trichloromethyl-(4'-methoxystyryl)-6-triazine.

As the oximes, for example, 3-benzoyloxyiminobutan-2-one, 3-propionyloxyiminobutan-2-one, 2-acetoxyiminopentan-3-one, 2-acetoxyimino-1-phenylpropan-1-one, 3-(4-toluenesulfonyloxy)iminobutane-2-one,and 2-ethoxycarbonyloxyimino-1-phenylpropane-1,2-octanedione 1-[4-(phenylthio)-2-(O-benzoyloxime)], and ethanone 1-[9-ethyl-6-(2-methylbenzoyl)-carbazole-3-yl]-1-(O-acetyloxi me).

According to one embodiment of the present invention, the proportion of the photoradical polymerization initiator blended in the curable composition is such that the photoradical polymerization initiator is within the range of from 0.01 to 20 parts by mass and more preferably from 0.1 to 10 parts by mass with respect to 100 parts by mass of the curable composition. A cured product that is further excellent in heat resistance can be obtained by allowing the photoradical polymerization initiator to be contained in this range.

### Epoxy Compound Different from Alicyclic Epoxy Acrylate Compound of Formula (1-2) and/or Epoxy Group-Containing (Meth)acrylic Polymer Comprising Polymerization Unit Represented by Formula (2)

According to one embodiment of the present invention, the other epoxy compound contained in the curable composition is not particularly limited as long as it is a compound other than the above-described alicyclic epoxy acrylate compound represented by Formula (1-2) and/or an epoxy group-containing (meth)acrylic polymer comprising the polymerization unit represented by Formula (2), which has one or more epoxy groups in the molecule.

According to one embodiment of the present invention, examples of the other epoxy compound contained in the curable composition include glycidyl ether-type epoxide, glycidyl ester-type epoxide, glycidyl amine-type epoxide, and alicyclic epoxide. At least one selected from the group consisting of glycidyl ether-type epoxide, glycidyl amine-type epoxide, and alicyclic epoxide is preferred. In addition, the other epoxy compound may be an epoxy resin in which glycidyl ether-type epoxide, glycidyl ester-type epoxide, glycidyl amine-type epoxide, alicyclic epoxide, and the like are polymerized. Further, the above-described curable composition may contain one kind or two or more kinds of other epoxy compounds.

As glycidyl ether type epoxide, they are bisphenol A diglycidyl ether, bisphenol F diglycidyl ether, bisphenol S diglycidyl ether, tetramethylbiphenol diglycidyl ether, hydrogenated bisphenol A diglycidyl ether, glycidyl ether of dihydric phenol such as brominated bisphenol A diglycidyl ether, dihydroxynaphthyl cresol triglycidyl ether, tris (hydroxyphenyl) methane triglycidyl ether, dinaphthyltriol triglycidyl ether, phenol novolac glycidyl ether, and cresol novolac glycidyl ether, for example,xylylene skeleton-containing phenol novolac glycidyl ether, dicyclopentadiene skeleton-containing phenol novolac glycidyl ether, Biphenyl skeleton-containing phenol novolac glycidyl ether, terpene novolac glycidyl ether, bisphenol A novolac glycidyl ether, bisphenol S novolac glycidyl ether, bisphenol AP novolac glycidyl ether, bisphenol E novolac glycidyl ether, bisphenol Z novolac glycidyl ether, tetramethyl bisphenol A novolac glycidyl ether, tetramethyl bisphenol F novolac glycidyl ether,dimethyl bisphenol F novolac glycidyl ether, tetramethyl bisphenol S novolac glycidyl ether, Dimethylbisphenol S novolac glycidyl ether, tetramethyl-4,4'-biphenol novolac glycidyl ether, tris-hydroxyphenyl methane novolac glycidyl ether, resorcinol novolac glycidyl ether, hydroquinone volac glycidyl ether, diisopropylidene novolac glycidyl ether, 1,1-di-4-hydroxyphenyl fluorene novolac glycidyl ether, phenolated polybutadiene novolac glycidyl ether, ethylphenol novolac glycidyl ether, butylphenol novolac glycidyl ether,octylphenol novolac glycidyl ether, naphthol novolac glycidyl ether, glycidyl ether of polyhydric phenol such as hydrogenated phenol novolac glycidyl ether, ethylene glycol diglycidyl ether, Propylene glycol diglycidyl ether, tetramethylene glycol diglycidyl ether, 1,6-hexanediol diglycidyl ether, cyclohexanedimethylol diglycidyl ether, and polyethylene glycol diglycidyl ether, Examples thereof include glycidyl ethers of dihydric alcohols such as polypropylene glycol diglycidyl ether, trimethylolpropane triglycidyl ether, glycerin triglycidyl ether, glycidyl ether of polyhydric alcohols such as pentaerythritol tetraglycidyl ether, sorbitol hexaglycidyl ether, and polyglycerin polyglycidyl ether, and triglycidyl isocyanurate.

Examples of the glycidyl ester type epoxide include glycidyl esters of carboxylic acids such as glycidyl methacrylate, diglycidyl ester phthalate, diglycidyl ester isophthalate, diglycidyl ester terephthalate, diglycidyl ester cyclohexanedicarboxylate, and triglycidyl ester trimet acid, and polyepoxides of glycidyl ester type.

As a glycidylamine type epoxide, they are N,N-diglycidylaniline, N,N-diglycidyl toluidine, N,N,N', N'-tetraglycidyldiaminodiphenylmethane, N,N,N', N'-tetraglycidyldiaminodiphenylsulfone, N,N,N', and N'-tetraglycidyldiethyldiphenylmethane, bis (N,N-diglycidylaminocyclohexyl) methane (N,N,N', hydride of N'-tetraglycidyldiaminodiphenylmethane), N,N,N', N'-tetraglycidyl-1,3-(bisaminomethyl)cyclohexane (N,N,N', hydrogenated product of N'-tetraglycidylxylyldiamine),and glycidyl heterocyclic amines such as triglycidyl-p-aminophenol, N-glycidyl-4-glycidyloxypyrrolidone, and the like.

As alicyclic epoxides, vinylcyclohexene dioxide, limonene dioxide, dicyclopentadiene dioxide, bis (2,3-epoxycyclopentyl) ether, ethylene glycol bisepoxydicyclopentyl ether, 3,4-epoxy-6-methylcyclohexylmethyl 3', 4'-epoxycyclohexanecarboxylate,3,4-epoxy-1-methylcyclohexyl 3,4-epoxy-1-methylhexanecarboxylate, 3,4-epoxy-3-methylhexanecarboxylate, 3,4-epoxy-5-methylcyclohexyl methyl 3,4-epoxy-5-methylcyclohexanecarboxylate, 2-(3,4-epoxycyclohexyl-5,5-spiro-3,4-epoxy)cyclohexane, methylenebis(3,4-epoxycyclohexane), (3,3 ', 4,4'-diepoxy)bicyclohexyl, 1,2-epoxy-(2-oxiranyl)cyclohexane adducts of 2,2-bis(hydroxymethyl)-1-butanol, tetrahydroindenediepoxide, 3,4-epoxymethylmethacrylate, and the like, and preferably is 3,4-epoxycyclohexylmethyl 3,4-epoxycyclohexanecarboxylate, 3,4-epoxycyclohexylmethyl methacrylate.

According to one embodiment of the present invention, the proportion of the epoxy compound that is different from the alicyclic epoxy acrylate compound of Formula (1-2) blended in the curable composition is such that the epoxy compound is within the range of from 10 to 90 parts by mass and more preferably from 20 to 80 parts by mass with respect to 100 parts by mass in total of the curable composition from the viewpoint of heat resistance of the cured product.

According to one embodiment of the present invention, the content ratio of the alicyclic acrylate-type polymerizable monomer comprising the alicyclic epoxy acrylate compound of Formula (1-2) and the epoxy compound that is different from the alicyclic epoxy acrylate compound (the alicyclic acrylate-type polymerizable monomer comprising the alicyclic epoxy acrylate compound of Formula (1-2) : the epoxy compound that is different from the alicyclic epoxy acrylate compound) in the curable composition is preferably from 1:0.1 to 1:10 and more preferably from 1:0.5 to 1:5 on a mass basis.

According to one embodiment of the present invention, the content ratio of the epoxy group-containing (meth)acrylic polymer comprising the polymerization unit represented by Formula (2) and the other epoxy compound (the epoxy group-containing (meth)acrylic polymer : the other epoxy compound) in the curable composition is preferably from 1:0.1 to 1:10 and more preferably from 1:0.5 to 1:5 on a mass basis.

### Radical-polvmerizable Compound Different from Alicyclic Epoxy Acrylate Compound of Formula (1-2) and/or Radical-polymerizable Compound Containing Polymerization Unit of Formula (3)

According to one embodiment of the present invention, the other radical-polymerizable compound contained in the curable composition is not particularly limited as long as it is a compound other than the above-described alicyclic epoxy acrylate compound represented by Formula (1-2) and/or a radical-polymerizable compound comprising the polymerization unit of Formula (3), which has one or more double bonds in the molecule.

According to one embodiment of the present invention, examples of the other radical-polymerizable compound contained in the curable composition include (meth)acrylic acid esters, styrenes, (meth)acrylamides, vinyl ethers, maleimides, and maleic anhydrides. (Meth)acrylic acid esters are preferred. In addition, the above-described curable composition may contain two or more kinds of the above-described radical-polymerizable compounds. Further, the above-described curable composition may contain one kind or two or more kinds of the above-described radical-polymerizable compounds.

As (meth) acrylic esters, for example, (meth) acrylic acid methyl, (meth) acrylic acid propyl, chlorethyl (meth) acrylate, 2-hydroxyethyl (meth) acrylate, trimethylolpropane mono (meth) acrylate, benzyl (meth) acrylate, phenoxyethyl (meth) acrylate, furfuryl (meth) acrylate, 2-(metha)acryloyloxyethyl succinate,(meth) acrylic acid 2-carboxyethyl, epichlorohydrin (ECH) modified glycerol tri (meth) acrylate, ethylene oxide (EO) modified glycerol tri (meth) acrylate, pentaerythritol triacrylate, Pentaerythritol tetraacrylate, EO-modified phosphoric acid triacrylate, trimethylolpropane tri (meth) acrylate, caprolactone-modified trimethylolpropane tri (meth) acrylate, PO-modified trimethylolpropane tri (meth) acrylate, tris (acryloxyethyl) isocyanurate, dipentaerythritol hexa (meth) acrylate, caprolactone-modified dipentaerythritol hexa (meth) acrylate, dipentaerythritol hydroxypenta (meth) acrylate,alkyl-modified dipentaerythritol penta(meth)acrylate, dipentaerythritol poly(meth)acrylate, alkyl-modified dipentaerythritol tri(meth)acrylate, Examples thereof include pentaerythritol ethoxytetra (meth) acrylate, pentaerythritol tetra (meth) acrylate, and the like, and are preferably methyl methacrylate, pentaerythritol triacrylate, pentaerythritol tetraacrylate, and dipentaerythritol hexaacrylate.

Examples of the styrene include styrene, α-methylstyrene, 2-methylstyrene, 3-methylstyrene, 4-methylstyrene, 2-aminostyrene, 3-aminostyrene, 4-aminostyrene, and indene.

Examples of the (meth) acrylamides include acrylamide, N-alkylacrylamide (having 1 to 3 carbon atoms as an alkyl group, for example, a methyl group, an ethyl group, a propyl group), N,N-dialkylacrylamide (having 1 to 6 carbon atoms as an alkyl group), N-hydroxyethyl-N-methylacrylamide, and N-2-acetamidoethyl-N-acetylacrylamide.

As vinyl ethers, they are n-propylvinyl ether, i-propylvinyl ether, hydroxyethylvinyl ether, n-butylvinyl ether, i-butylvinyl ether, tert-butylvinyl ether, n-pentylvinyl ether, i-pentylvinyl ether, neopentyl vinyl ether, tert-pentyl vinyl ether, 1-methylbutylvinyl ether, 2-methylbutylvinyl ether, 1,2-dimethylpropylvinyl ether, and the like, for example.

Examples of the maleimides include maleimide and N-methylmaleimide.

Examples of the maleic anhydride include maleic anhydride and 2-methylmaleic anhydride.

According to one embodiment of the present invention, the proportion of the radical-polymerizable compound that is different from the alicyclic epoxy acrylate compound of Formula (1-2) and/or the radical-polymerizable compound comprising the polymerization unit of Formula (3) blended in the curable composition is such that the radical-polymerizable compound is within the range of from 1 to 60 parts by mass and more preferably from 5 to 40 parts by mass with respect to 100 parts by mass in total of the curable composition from the viewpoint of heat resistance of the cured product.

According to one embodiment of the present invention, the content ratio of the alicyclic acrylate-type polymerizable monomer comprising the alicyclic epoxy acrylate compound of Formula (1-2) or the radical-polymerizable compound comprising the polymerization unit of Formula (3) and the other radical-polymerizable compound (the alicyclic acrylate-type polymerizable monomer comprising the alicyclic epoxy acrylate compound of Formula (1-2) or the radical-polymerizable compound comprising the polymerization unit of Formula (3) : the other radical-polymerizable compound) in the curable composition is preferably from 1:0.01 to 1:10 and more preferably from 1:0.1 to 1:1 on a mass basis.

### Curing Accelerator

According to one embodiment of the present invention, the curable composition may further contain a curing accelerator. The Curing accelerators include triphenylphosphine, triphenylbenzylphosphonium tetraphenylborate, tetrabutylphosphonium diethylphosphodithioate, tetrabutylphosphonium acetate, tetra-n-butylphosphonium bromide, tetra-n-butylphosphonium tetrafluoroborate, tetra-n-butylphosphonium tetraphenylborate, methyltriphenylphosphonium bromide, ethyltriphenylphosphonium phosphonium bromide, ethyltriphenylphosphonium phosphonium phosphate, n-butyltriphenylphosphonium bromide, benzyltriphenylphosphonium chloride, Phosphines such as tetraphenylphosphonium tetraphenylborate and their quaternary salts, 2-ethyl-4-methylimidazole, 1,2-dimethyimidazole, 1-benzyl-2-phenylimidazole, 2-methylimidazole, 1-(2-cyanoethyl)-2-ethyl-4-methylimidazole, 2,4-diamino-6-[2-methylimidazolyl-(1)]ethyl-triazine, 2-phenylimidazoline, 2,3-dihydro-1H-pyrrolo[1,2-a]benzimidazole, 3-dimethylamine, and other quaternamide salts ultra-strong basic organic compounds such as 1,8-diazabicyclo(5,4,0)undecene-7 and 1,5-diazabicyclo(4,3,0)nonene-5, zinc octylate, zinc laurate, and zinc stearate, Organocarboxylate metal salts such as tin octylate, metal-organochelate compounds such as benzoylacetone zinc chelates, dibenzoylmethane zinc chelates and acetoacetate ethylzinc chelates, tetra-n-butylsulfonium O,O-diethylphosphodithionate (tetrabutylphosphonium diethylphosphorodithioate), and the like, preferably imidazoles, tetra-n-butylsulfonium O,O-diethylphosphorodithionate The curable composition may contain one kind, or two or more kinds of the curing accelerators as described above.

According to one embodiment of the present invention, the proportion of the curing accelerator blended in the curable composition is such that the curing accelerator is within the range of from 0.1 to 10 parts by mass and more preferably from 0.5 to 5 parts by mass with respect to 100 parts by mass of the curable composition.

### Photosensitizer

According to one embodiment of the present invention, the curable composition may further contain a photosensitizer. Photocurability can be improved by allowing the curable composition to contain a photosensitizer.
As a photosensitizer, they are N,N-dimethylaminobenzoic acid ethyl ester, N,N-dimethylaminobenzoic acid isoamyl ester, pentyl-4-dimethylaminobenzoate, triethylamine, and triethanolamine, Examples thereof include tertiary amines such as pyralizones, anthracenes, coumarins, xanthones, and thioxanthones such as 2,4-diethylthioxanthone, and the like, and are preferably thioxanthones, and more preferably 2,4-diethylthioxanthone.

The above-described curable composition may contain one kind, or two or more kinds of the photosensitizers as described above.

According to one embodiment of the present invention, the proportion of the photosensitizer blended in the curable composition is such that the photosensitizer is within the range of from 0.1 to 10 parts by mass and more preferably from 0.5 to 5 parts by mass with respect to 100 parts by mass in total of the curable composition.

### Reactive Diluent

According to one embodiment of the present invention, the curable composition may further contain a reactive diluent in order to reduce the viscosity. Examples of the reactive diluent include a monoepoxy compound produced by the method described in WO2017/159637, butyl glycidyl ether, 2-ethylhexyl glycidyl ether, glycidyl ether of a mixture of C12 and C13 alcohols, and 1,2-epoxy-4-vinylcyclohexane. The curable composition may contain one kind, or two or more kinds of the reactive diluents as described above. The mixing ratio of the reactive diluent may be adjusted as appropriate such that the curable composition containing the reactive diluent has a desired viscosity.

### Oxetane Compound

According to one embodiment of the present invention, the curable composition may further contain an oxetane compound. As an oxetane compound, they are 1,4-bis[(3-ethyl-3-oxetanylmethoxy)methyl]benzene, 3-ethyl-3-(phenoxymethyl)oxetane, di[(3-ethyl-3-oxetanyl)methyl]ether, 3-ethyl-3-(cyclohexyloxymethyl)oxetane, and phenol novolac oxetane, for example 1,3-bis[(3-ethyloxetan-3-yl)]methoxybenzene, oxetanyl silicicate, bis[1-ethyl(3-oxetanyl)]methyl ether, 4,4'-bis(3-ethyl-3-oxetanylmethoxy)biphenyl, ethylene glycol (3-ethyl-3-oxetanylmethyl)ether, Examples thereof include diethylene glycolbis(3-ethyl-3-oxetanylmethyl)ether, bis(3-ethyl-3-oxetanylmethyl)diphenoate, trimethylol propane tris(3-ethyl-3-oxetanylmethyl)ether, pentaerythritol tetrakis(3-ethyl-3-oxetanylmethyl)ether, and phenol novolac type oxetane.
The above-described curable composition may contain one kind, or two or more kinds of the oxetane compounds as described above.

The proportion of the oxetane compound blended in the curable composition is such that the oxetane compound is within the range of from 1 to 90 parts by mass and more preferably from 5 to 85 parts by mass with respect to 100 parts by mass of the curable composition.

### Vinyl Ether Compound

According to one embodiment of the present invention, the curable composition may further contain a vinyl ether compound. As a vinyl ether compound, they are monofunctional vinyl ether, such as methyl vinyl ether, ethyl vinyl ether, and butyl vinyl ether, for example, Butanediol divinyl ether, cyclohexanedimethanol divinyl ether, cyclohexanediol divinyl ether, trimethylolpropanetrivinyl ether, pentaerythritol tetravinyl ether, glycerol trivinyl ether, triethylene glycol divinyl ether, and diethylene glycol divinyl ether, hydroxyethylvinyl ether, cyclohexanedimethanol monovinyl ether, cyclohexanediol monovinyl ether,9-hydroxynonyl vinyl ether, propylene glycol monovinyl ether, neopentyl glycol monovinyl ether, Glycerol divinyl ether, trimethylolpropanedivinyl ether, trimethylolpropanemonovinyl ether, pentaerythritol divinyl ether, pentaerythritol trivinyl ether, diethylene glycol monovinyl ether, tetraethylene glycol monovinyl ether, tricyclodecanediol monovinyl ether, vinyl ether compounds having hydroxyl groups such as tricyclodecanedimethanol monovinyl ether and acrylic acid 2-(2-vinyloxyethoxy)ethyl,Examples thereof include vinyl ethers having different functional groups such as 2-(2-vinyloxyethoxy)ethyl methacrylate. The above-described curable composition may contain one kind, or two or more kinds of the vinyl ether compounds as described above.

The proportion of the vinyl ether compound blended in the curable composition is such that the vinyl ether compound is within the range of from 1 to 90 parts by mass and more preferably from 5 to 85 parts by mass with respect to 100 parts by mass in total of the curable composition.

### Compound Containing Hydroxyl Group

According to one embodiment of the present invention, the curable composition may further contain a compound containing a hydroxyl group. Incorporation of a compound containing a hydroxyl group into the curable composition allows a moderate curing reaction to proceed. Examples of the compound containing a hydroxyl group include ethylene glycol, diethylene glycol, and glycerin. The curable composition may contain one kind, or two or more kinds of the compounds containing a hydroxyl group, such as those described above.

The proportion of the compound containing a hydroxyl group blended in the curable composition is such that the compound containing a hydroxyl group is within the range of from 0.1 to 10 parts by mass and more preferably from 0.2 to 8 parts by mass with respect to 100 parts by mass of the curable composition.

### Solvent/Others

According to one embodiment of the present invention, the curable composition may further contain a solvent. Examples of the solvent include methyl ethyl ketone, ethyl acetate, toluene, methanol, and ethanol.

According to one embodiment of the present invention, the curable composition may contain various types of components to the extent that the properties of the composition are not impaired. Examples of the components include fillers, silane coupling agents, mold release agents, flame retardants, antioxidants, antifoaming agents, photostabilizers, coloring agents such as pigments and dyes, plasticizers, pH adjusting agents, coloration inhibitors, matting agents, deodorants, weather resistant agents, antistatic agents, yarn friction reducing agents, slip agents, and ion exchangers.

### Method of Producing Curable Composition

According to one embodiment of the present invention, the curable composition can be produced in accordance with technical common knowledge widely known to those skilled in the art, and the method of producing the curable composition and the components to be further included in the curable composition can be selected as appropriate.

According to a preferred embodiment of the present invention, in the method of preparing a curable composition, for example, a curable composition can be produced by kneading or mixing an alicyclic acrylate-type polymerizable monomer comprising the alicyclic epoxy acrylate compound of Formula (1-2) and optionally the above-described components and other components to be added as appropriate.

The kneading or mixing method is not particularly limited, and can be mixed, for example, using a planetary mixer, a biaxial extruder, a mixing device such as a heat roller or a kneader, or a kneader or the like.

### Cured Product and Method of Producing the Same

According to one embodiment of the present invention the cured product is obtained by curing the above described curable composition according to the present invention. The method of curing the curable composition is not particularly limited, and the composition can be cured by heating or irradiation of light, as appropriate.

### Conditions for Curing

In cases where the curable composition is cured by heating, the heating of the curable composition is preferably carried out in multiple stages. This allows for a sufficient curing reaction to proceed. For example, the curing reaction can be carried out by performing a first heating at a temperature of from 60°C to 110°C for 10 to 150 minutes, a second heating at 120°C to 170°C for 10 to 150 minutes, a third heating at 180°C to 220°C for 10 to 150 minutes, and a fourth heating at 230°C to 250°C for 10 to 300 minutes. Alternatively, the curing reaction can also be carried out, for example, by performing a first heating at a temperature of from 60°C to 90°C for 10 to 150 minutes, a second heating at 100°C to 120°C for 10 to 150 minutes, and a third heating at 130°C to 160°C for 10 to 150 minutes, a fourth heating at 170°C to 220°C for 10 to 150 minutes, and a fifth heating at 230°C to 250°C for 10 to 150 minutes. However, curing is not limited thereto and is preferably carried out by changing the conditions as appropriate in consideration of the blending proportion of the alicyclic acrylate-type polymerizable monomer comprising the alicyclic epoxy acrylate compound of Formula (1-2), properties of other compounds contained in the curable composition, and the like.

In cases where the curable composition is cured by the irradiation of an active energy ray, such as a visible ray, UV ray, X-ray, or electron beam, the type of the active energy ray used and the conditions for irradiation are preferably selected as appropriate, depending on the composition of the curable composition. In one embodiment, it is preferred that the irradiation of UV light be carried out such that the cumulative irradiation dose (accumulated amount of light) is adjusted within the range of from 100 to 5,000 mJ/cm². When the cumulative irradiation dose on the curable composition is adjusted within the above described numerical range, it is possible to allow active species derived from the photo-cationic polymerization initiator to be generated sufficiently.

### Properties of Cured Product

According to one embodiment of the present invention, heat resistance of the cured product can be evaluated by measuring at least one selected from the glass-transition temperature and a specific thermogravimetric loss temperature. The glass-transition temperature and a specific thermogravimetric loss temperature are preferably high from the viewpoint of imparting heat resistance.

According to one embodiment of the present invention, the curable composition comprising the alicyclic acrylate-type polymerizable monomer comprising the alicyclic epoxy acrylate compound represented by Formula (1-2), the epoxy group-containing (meth)acrylic polymer containing the polymerization unit of Formula (2), or the radical-polymerizable compound containing the polymerization unit of Formula (3) can remarkably improve heat resistance as compared to a curable composition which comprises none of the alicyclic acrylate-type polymerizable monomer comprising the alicyclic epoxy acrylate compound represented by Formula (1-2), the epoxy group-containing (meth)acrylic polymer containing the polymerization unit of Formula (2), and the radical-polymerizable compound containing the polymerization unit of Formula (3).

The glass-transition temperature can be measured by at least one selected from differential scanning calorimetry (DSC) and thermomechanical analysis (TMA).

Glass-transition temperature measurement by DSC can be conveniently carried out using a commercially available differential scanning calorimeter (e.g., trade name: DSC7000X manufactured by Hitachi High-Tech Corporation). The glass-transition temperature of the cured product measured by DSC can be 200°C or more, and is preferably 230°C or more. The upper limit thereof is not particularly limited, but it is preferably 400°C or less.

Glass-transition temperature measurement by TMA can be conveniently carried out using a commercially available thermomechanical analyzer (e.g., trade name: TMA7100 manufactured by Hitachi High-Tech Corporation). Here, the glass-transition temperature measured by TMA can be obtained by using a thermomechanical analyzer to increase the temperature from 20°C to 300°C at a rate of 10°C/min to create a TMA curve and extrapolating the curves before and after the inflection point of the TMA curve due to the glass transition.

The glass-transition temperature measured by TMA may vary generally due to the formulation of a curable composition, but it can be, for example, 100°C or more and is preferably 200°C or more. The upper limit thereof is not particularly limited, but it is preferably 400°C or less. The glass-transition temperature measured by TMA can be 200°C or more in cases where a curable composition 1 is used and it can be 100°C or more in cases where a curable composition 5 is used.

The specific thermogravimetric loss temperature can be evaluated by, for example, measuring the temperature when the rate of thermogravimetric loss of the cured product is 1%, 5%, 10%, or 20%. Here, for example, the temperature when the rate of thermogravimetric loss is 10% (10% thermogravimetric loss temperature) is the temperature at which the weight of the cured product is reduced by 10% compared to the start of measurement when increasing the temperature from 30°C to 400°C at a rate of 20°C/min using a differential thermal thermogravimetric analysis system. Such measurement can be conveniently carried out using a commercially available differential thermal thermogravimetric analysis system (trade name: TG/DTA7300 manufactured by Hitachi High-Tech Corporation).

The 1% thermogravimetric loss temperature of a cured product may vary generally due to the formulation of a curable composition, but it can be, for example, 70°C or more and is preferably 280°C or more. The upper limit thereof is not particularly limited, but it is preferably 400°C or less. The 1% thermogravimetric loss temperature of the cured product can be 280°C or more in cases where a curable composition 1 is used and it can be 70°C or more in cases where a curable composition 5 is used.

The 5% thermogravimetric loss temperature of a cured product may vary generally due to the formulation of a curable composition, but it can be, for example, 150°C or more and is preferably 230°C or more, more preferably 280°C or more, still more preferably 290°C or more, and yet more preferably 320°C or more. The upper limit thereof is not particularly limited, but it is preferably 400°C or less. The 5% thermogravimetric loss temperature of the cured product can be 320°C or more in cases where a curable composition 2 is used, 235°C or more in cases where a curable composition 3 is used, 290°C or more in cases where a curable composition 4 is used, and 150°C or more in cases where a curable composition 5 is used.

The 10% thermogravimetric loss temperature of a cured product may vary generally due to the formulation of a curable composition, but it can be, for example, 180°C or more, and it is preferably 265°C or more and more preferably 320°C or more. The upper limit thereof is not particularly limited, but it is preferably 450°C or less. The 10% thermogravimetric loss temperature of the cured product can be 265°C or more in cases where a curable composition 3 is used, 320°C or more in cases where a curable composition 4 is used, and 180°C or more in cases where a curable composition 5 is used.

According to one embodiment of the present invention, transparency of the cured product can be evaluated by measuring at least one selected from the total luminous transmittance and the yellow index. From the viewpoint of improving transparency, the total luminous transmittance is preferably high and the yellow index is preferably low.

Measurement of total luminous transmittance of the cured product can be conveniently carried out using a commercially available haze meter (trade name: NDH5000 manufactured by NIPPON DENSHOKU INDUSTRIES CO., LTD.) in accordance with the method described in JIS K 7361-1.

The total luminous transmittance of a cured product (3 mm in thickness) may vary generally due to the formulation of a curable composition, but it can be, for example, 50% or more and is preferably 70% or more. The upper limit thereof is not particularly limited, but it is preferably 100% or less. The total luminous transmittance of the cured product can be 50% or more in cases where a curable composition 1 is used and it can be 60% or more in cases where a curable composition 2 is used.

Measurement of yellow index of the cured product can be conveniently carried out using a commercially available spectrophotometer (trade name: SD6000 manufactured by NIPPON DENSHOKU INDUSTRIES CO., LTD.) in accordance with the method described in JIS K 7373-2006.

The yellow index of a cured product (3 mm in thickness) may vary generally due to the formulation of a curable composition, but it can be, for example, 100 or less and is preferably 50 or less. The lower limit thereof is not particularly limited, but it is preferably 0 or more. The yellow index of the cured product can be 100 or less in cases where a curable composition 1 is used, and it can be, for example, 70 or less, preferably 60 or less, and more preferably 50 or less in cases where a curable composition 2 is used.

### Applications of Cured Product

According to one embodiment of the present invention, specific examples of the application of the cured product include: adhesive agents; tacky materials; coating materials for coating on substrates such as metals, resin films, glass, paper and wood, surface protective films for semiconductor devices and organic thin film elements (for example, organic electroluminescent elements and organic thin film solar cell elements), hard coating agents, anti-fouling films and antireflection films; various types of optical members such as lenses, prisms, filters, image display materials, lens arrays, sealing materials and reflector materials for optical semiconductor devices, sealing materials for semiconductor devices, optical waveguides, light guide plates, light diffusion plates, diffraction elements and optical adhesive agents; materials such as casting materials, interlayer insulators, insulating films for printed alignment substrates and fiber-reinforced composite materials; and resist materials.

### EXAMPLES

The present invention will now be specifically described by way of Examples and Comparative Examples. However, the present invention is in no way limited by these Examples and Comparative Examples.

### <Component (A): Alicyclic epoxy acrylate compound>

The following alicyclic epoxy acrylate-type polymerizable monomer (A1) was synthesized and used as a component (A). (Hereinafter, the alicyclic epoxy acrylate-type polymerizable monomer (A1) is also simply referred to as "epoxy acrylate compound (A1).")

### Preparation Example 1: Synthesis of Alicyclic Epoxy Acrylate-type Polymerizable Monomer (A1) (tricyclopentadiene-epoxy methacrylate: TCPD-EM) Preparation Example 1-1: Synthesis of Alicyclic Acrylate-type Polymerizable Monomer (tricyclopentadiene- methacrylate: TCPD-M)

Into a reaction vessel equipped with a thermometer, an agitator, and a reflux tube, 120 g of a diolefin compound represented by Formula (1-a-1), 1.2 L of isopropyl ether, 1.96 g of iron (III) chloride, and 56.2 mL of methacrylic acid were charged. Then, 3.2 mL of trifluoromethanesulfonic acid was slowly added at room temperature, and the mixture in the reaction vessel was continuously agitated under reflux conditions for 25 hours. Then, the mixture was allowed to cool to room temperature and washed with 1 L of saturated aqueous sodium hydrogen carbonate, following which the obtained aqueous layer was extracted twice with 1 L of isopropyl ether. The obtained organic layer was mixed, purified by a silica gel column (Silica Gel 60 (spherical) manufactured by KANTO CHEMICAL CO., INC.), and then concentrated by an evaporator to obtain 170 g of a yellow liquid.

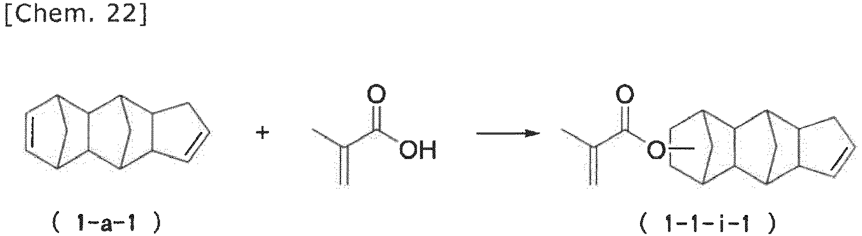

The ¹H-NMR spectrum and the ¹³C-NMR spectrum were measured for the obtained yellow liquid. Specifically, the measurement was carried out under the following conditions.
[¹H-NMR measurement conditions]
   Measurement system: DD2 (Agilent Technologies)
   Solvent: CDCl₃
   Pulse angle: 45° pulse
   Sample concentration: 1% by mass
   Number of accumulation times: 64 times
[¹³C-NMR measurement conditions]
   Measurement system: DD2 (Agilent Technologies)
   Solvent: CDCl₃
   Pulse angle: 45° pulse
   Sample concentration: 10% by mass
   Number of accumulation times: 1024 times

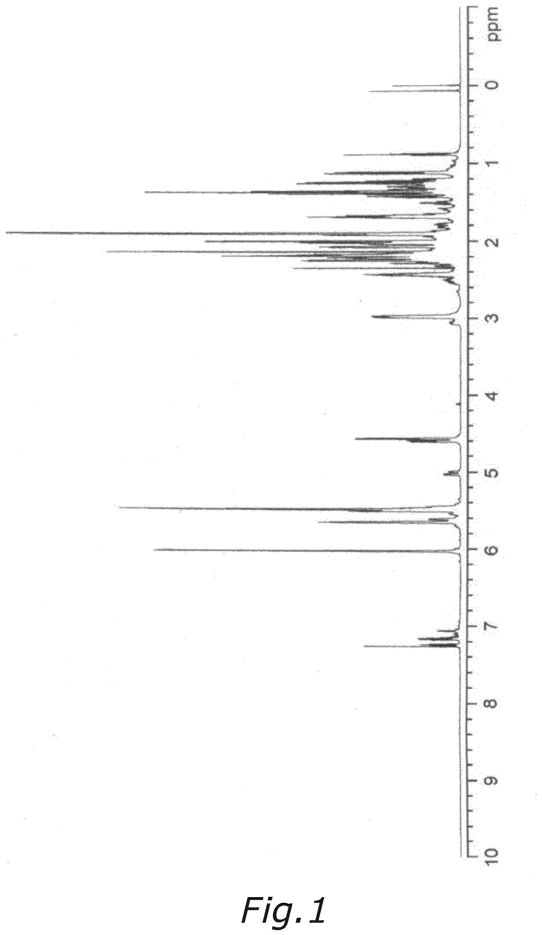

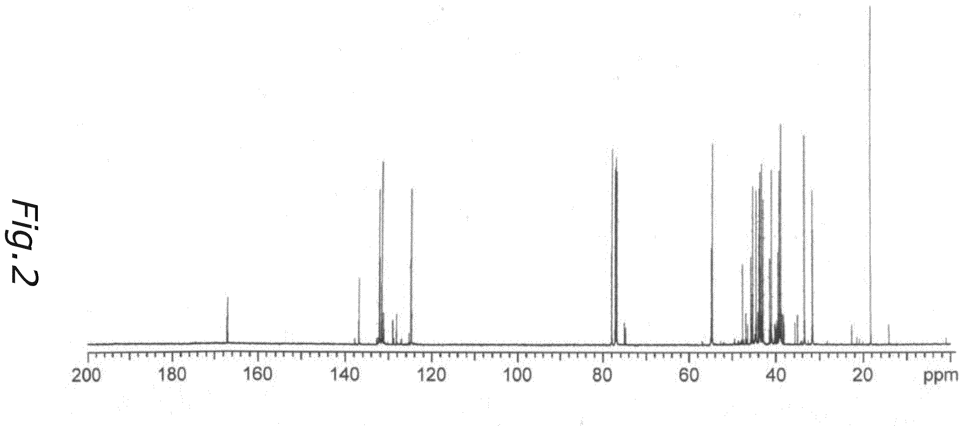

FIG. 1 shows the ¹H-NMR spectrum of the obtained yellow liquid and FIG. 2 shows the ¹³C-NMR spectrum of the same.

A peak characteristic of the methacryl moiety was confirmed at 6.028 ppm and 5.658 ppm in ¹H-NMR and at 167.099 ppm (corresponding to carbonyl carbon) in ¹³C-NMR. In addition, a peak common in the olefin region was confirmed at from 131.221 to 132.148 ppm in ¹³C-NMR and at 5.481 ppm in ¹H-NMR.

(¹H NMR spectrum of the alicyclic acrylate-type polymerizable monomer (1-1-i-1)) ¹H NMR (600 MHz, CDCl₃) δ (ppm): 6.028 (s, 1H), 5.658 (m, 1H), 5.481 (m, 2H), 4.579 (m, 1H),0.871-2.992 (m, 19H).

(¹³C NMR spectrum of the alicyclic acrylate-type polymerizable monomer (1-1-i-1)) ¹³C NMR (125 MHz, CDCl₃) δ (ppm): 167.099, 136.839, 136.816, 132.134, 132.042, 131.605, 131.513, 131.206, 124.792, 124.762, 124.693, 78.041, 76.815, 54.838, 54.684, 47.696, 46.899, 45.711, 45.397, 44.592, 44.546, 44.102, 43.880, 43.703, 43.619, 43.351, 43.251, 43.029, 42.960, 41.466, 41.106, 39.581, 39.289, 39.205, 38.975, 38.899, 33.527, 33.496, 31.711, 31.650, 31.619, 18.239.

From the above-described ¹H NMR spectrum measurement values and the calculated values of ¹H chemical shift assumed from the structure of Formula (1-1-i-3) obtained using Chemdraw Professional 16 (manufactured by CambridgeSoft Corporation) as well as the above-described ¹³C NMR spectrum measurement values and the calculated values of ¹³C chemical shift assumed from the structure of Formula (1-1-i-3) obtained using Chemdraw Professional 16 (manufactured by CambridgeSoft Corporation), it was confirmed that the obtained yellow liquid contained the compound represented by Formula (1-1-i-3).

It was confirmed that the obtained yellow liquid contained the compound represented by Formula (1-1-i-2) as with the compound represented by Formula (1-1-i-3),

From the above, it was confirmed that the obtained yellow liquid was a mixture of the alicyclic acrylate-type polymerizable monomer represented by Formula (1-1-i-1), i.e., the compound represented by Formula (1-1-i-3), and the compound represented by Formula (1-1-i-2).

Further, mass spectrometry of the obtained yellow liquid was carried out by GC-MS. Specifically, the measurement was carried out under the following conditions.

### [GC-MS measurement conditions]

Measurement system: 7890A (Agilent Technologies)
Column: 19091S-936 (Agilent Technologies)
Column oven temperature: 80°C to 250°C
Carrier gas: N₂
Column flow rate: 1.4 mL/min
Injection mode: Split
Inlet temperature: 140°C
Injection volume: 2 µL
Transfer line temperature: 250°C
Ionization method: EI (electron ion method)
Detector: 5975C VL MSD (Agilent Technologies)
Scan range: m/z 50 to 1000
Sample preparation: 50 mg/mL (the solvent is acetone)

As a result, [M] = 284.1 corresponding to the theoretical structure was obtained by mass spectrometry by GC-MS. Therefore, it was also confirmed from mass spectrometry by GC-MS that the obtained yellow liquid was the target alicyclic acrylate-type polymerizable monomer represented by the above-described Formula (1-1-i-1).

### Preparation Example 1-2: Synthesis of Alicyclic Epoxy Acrylate-type Polymerizable Monomer (tricyclopentadiene-epoxy methacrylate: TCPD-EM) (A1)

Into a reaction vessel equipped with a thermometer, an agitator, and a reflux tube, 150 g of the alicyclic acrylate-type polymerizable monomer prepared in Preparation Example 1-1 above, 1.5 L of chloroform, and 143 g of m-chloroperoxybenzoic acid were charged and were continuously agitated at room temperature for 2 hours. Then, the precipitated solid was filtered off, and the filtrate was washed with 2 L of a 5% aqueous sodium sulfite solution, following which the obtained organic layer was washed three times with 1 L of saturated aqueous sodium hydrogen carbonate and washed once with 1 L of a saturated saline solution. Subsequently, the organic layer was purified by a silica gel column (Silica Gel 60 (spherical) manufactured by KANTO CHEMICAL CO., INC.) and concentrated by an evaporator to obtain 140 g of a white solid.

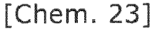

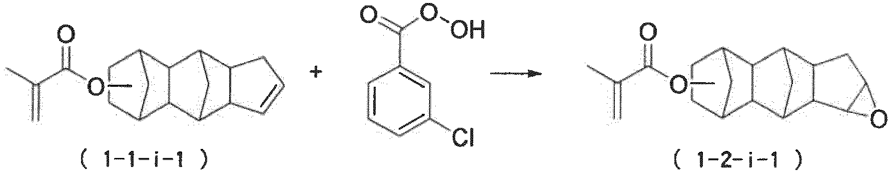

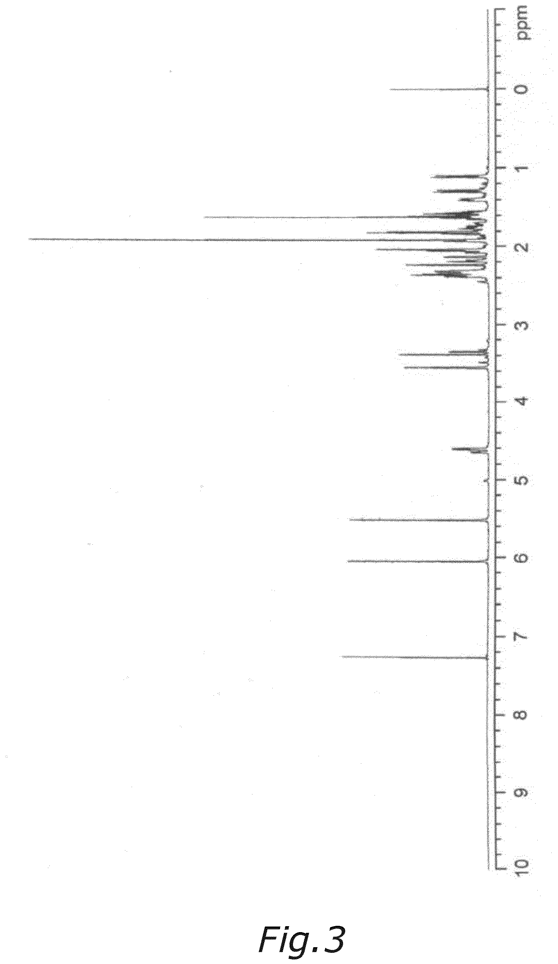

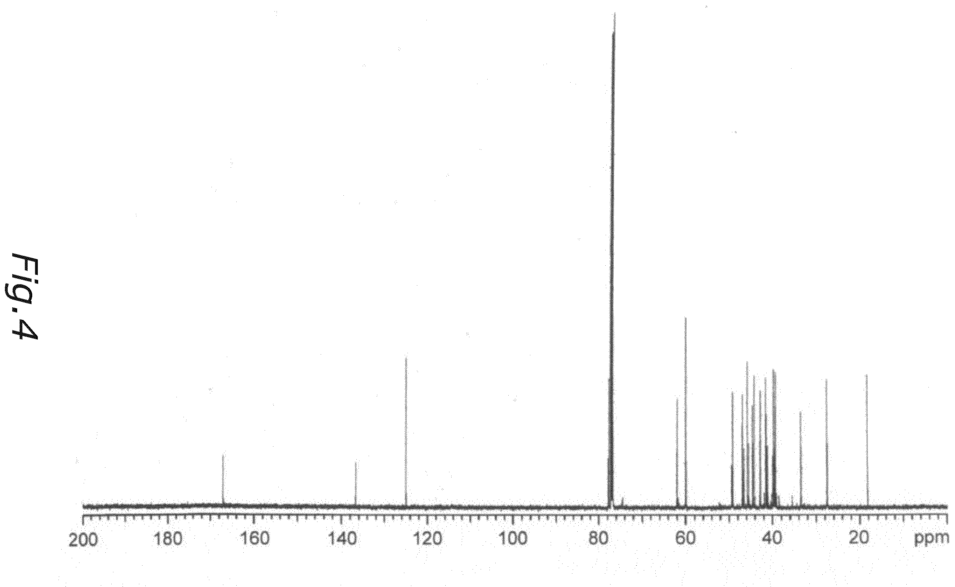

The obtained white solid was measured by ¹H-NMR, ¹³C-NMR, and GC-MS in the same manner as in Preparation Example 1-1. FIG. 3 shows the ¹H-NMR spectrum of the obtained white solid and FIG. 4 shows the ¹³C-NMR spectrum of the same.

As for the obtained white solid, the presence of a peak of the methacryl moiety was confirmed at 167.138 ppm (carbonyl carbon) in ¹³C-NMR and at 6.049 ppm and 5.520 ppm in ¹H-NMR. In addition, carbon and hydrogen peaks at the base of the epoxy were confirmed at 62.002 ppm and 59.972 ppm in ¹³C-NMR and at 3.556 ppm and 3.387 ppm in ¹H-NMR. Further, it was also confirmed that the 5.481 ppm peak from the double bond, which was confirmed for the acrylate compound of Formula (1-1-i-1) in Preparation Example 1-1, disappeared. In addition, [M] = 300.1 corresponding to the theoretical structure was obtained by mass spectrometry by GC-MS.

### (¹H NMR spectrum of the alicyclic epoxy acrylate-type polymerizable monomer (1-2-i-1))

¹H NMR (600 MHz, CDCl₃) δ (ppm): 6.049 (s, 1H), 5.520 (s, 1H), 4.601 (m, 1H), 3.556 (m, 1H), 3.387 (m, 1H), 1.099-2.378 (m, 19H).

### (¹³C NMR spectrum of the alicyclic epoxy acrylate-type polymerizable monomer (1-2-i-1))

¹³C NMR (125 MHz, CDCl₃) δ (ppm): 167.138, 136.685, 125.022, 77.796, 62.002, 59.972, 49.343, 49.159, 46.860, 46.569, 45.734, 45.719, 45.473, 44.554, 44.439, 44.117, 42.853, 42.761, 41.496, 41.404, 41.129, 39.857, 39.757, 39.719, 39.443, 39.297, 33.458, 33.404, 27.328, 18.224.

From the above-described ¹H NMR spectrum measurement values and the calculated values of ¹H chemical shift assumed from the structure of Formula (1-2-i-3) obtained using Chemdraw Professional 16 (manufactured by CambridgeSoft Corporation) as well as the above-described ¹³C NMR spectrum measurement values and the calculated values of ¹³C chemical shift assumed from the structure of Formula (1-2-i-3) obtained using Chemdraw Professional 16 (manufactured by CambridgeSoft Corporation), it was confirmed that the obtained white solid contained the compound of Formula (1-2-i-3).

It was confirmed that the obtained white solid contained the compound represented by Formula (1-2-i-2) as with the compound represented by Formula (1-2-i-3).

From the above, it was confirmed that the obtained white solid was a mixture of the alicyclic epoxy acrylate-type polymerizable monomer represented by Formula (1-2-i-1), i.e., the compound represented by Formula (1-2-i-3), and the compound represented by Formula (1-2-i-2).

The compounds and the like used in the following Examples and Comparative Examples are described below.

### <Component (A): Alicyclic epoxy acrylate compound>

The following was used as a component (A).
(A1): Alicyclic epoxy acrylate polymerizable monomer obtained by the method described in the above-described Preparation Example 1 (hereinafter, also simply referred to as "epoxy acrylate compound (A1)")

The following (CA1) to (CA3) were used as epoxy acrylate compounds for the Comparative Examples.
(CA1): 4-hydroxybutyl acrylate glycidyl ether (4HBAGE) represented by the following formula (manufactured by Mitsubishi Chemical Holdings Corporation) 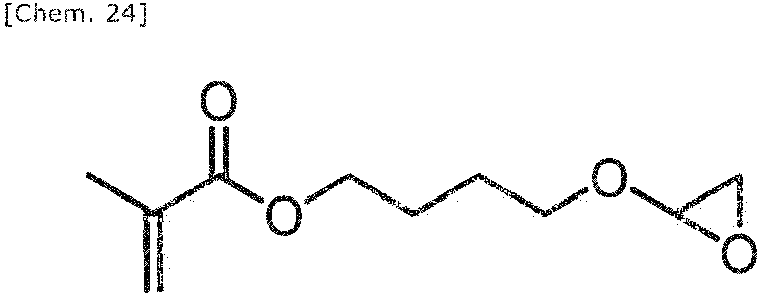
(CA2): 3, 4-Epoxy cyclohexylmethyl methacrylate (trade name: CYCLOMER M100 manufactured by Daicel Corporation) represented by the following formula 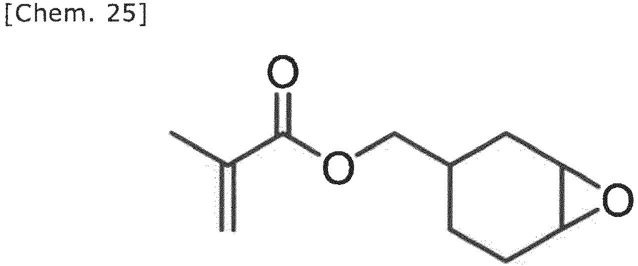
(CA3): Glycidyl methacrylate(GMA) (manufactured by Tokyo Chemical Industry Co., Ltd.) represented by the following formula

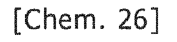

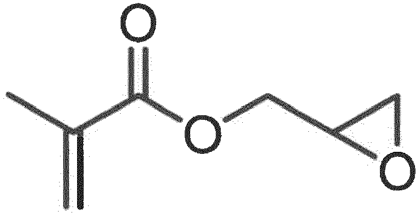

### <Component (B): Other epoxy compounds>

The following were used as components (B). (B1): 3, 4-Epoxy cyclohexylmethyl 3, 4-epoxy cyclohexanecarboxylate (trade name: CELLOXIDE 2021P (CEL2021P) manufactured by Daicel Corporation) (B2): 3, 4-Epoxy cyclohexylmethyl methacrylate (trade name: CYCLOMER M100 manufactured by Daicel Corporation)

### <Component (C): Radical-polymerizable compounds>

The following (C1) to (C3) were used as components (C).
(C1): Methyl methacrylate (MMA) (manufactured by Tokyo Chemical Industry Co., Ltd.)
(C2): Acrylic acid (manufactured by Tokyo Chemical Industry Co., Ltd.)
(C3): pentaerythritol triacrylate (trade name: LIGHT ACRYLATE PE-3A (acrylate containing pentaerythritol triacrylate as the main component) manufactured by Kyoeisha Chemical Co., Ltd.)
(C4): Pentaerythritol tetraacrylate (trade name: LIGHT ACRYLATE PE-4A manufactured by Kyoeisha Chemical Co., Ltd.)
(C5): Dipentaerythritol hexaacrylate (trade name: LIGHT ACRYLATE DPE-6A manufactured by Kyoeisha Chemical Co., Ltd.)

### <Component (D): Thermal cationic polymerization initiator>

The following was used as a component (D).
(D1): 4-Acetoxyphenyldimethylsulfonium hexafluoroantimonate (trade name: SI-150L manufactured by Sanshin Chemical Industry Co. Ltd.)

### <Component (E): Curing agent>

The following was used as a component (E1).
(E1): 3- or 4-Methylhexahydrophthalic anhydride (trade name: HN-5500 manufactured by Hitachi Chemical Company, Ltd.)

### <Component (F): Thermal radical polymerization initiators>

The following (F1) to (F3) were used as components (F).
(F1): t-Butylperoxy-2-ethylhexanoate (trade name: PERBUTYL (trademark) O manufactured by NOF CORPORATION)
(F2): 2, 2'-Azobisisobutyronitrile (AIBN) (manufactured by Tokyo Chemical Industry Co., Ltd.)
(F3): t-Butylperoxypivalate (PERBUTYL (trademark) PV)(manufactured by NOF CORPORATION)

### <Component (G): Curing Accelerator>

The following was used as a component (G).
(G1): Tetrabutylphosphonium diethyl phosphorodithioate (trade name: HISHICOLIN (trademark) PX-4ET manufactured by NIPPON CHEMICAL INDUSTRIAL CO., LTD.)

### <Component (H): Photo-cationic Polymerization Initiators>

The following were used as components (H).
(H1): 50% Propylene carbonate solution of diphenyl-4-(phenylthio)phenylsulfonium tris(pentafluoroethyl)trifluorophosphate (trade name: CPI-200K maufactured by San-Apro Ltd.)
(H2): Diphenyl-4-(phenylthio)phenylsulfonium tris(pentafluoroethyl)trifluorophosphate (trade name: CPI-210S maufactured by San-Apro Ltd.)

### <Component (I): Photoradical Polymerization Initiator>

The following was used as a component (I).
(I1): 2-Benzyl-2-dimethylamino-1-(4-morphorinophenyl)-butanone-1(dimethylamino morphorinophenyl benzyl butanone; MPBB) (manufactured by Tokyo Chemical Industry Co., Ltd.)

### <Other Components>

The following was used as a photosensitizer.

2, 4-Diethylthioxanthen-9-one (manufactured by Tokyo Chemical Industry Co., Ltd.)

The following was used as a catalyst.

Triphenylphosphine (Pph3) (manufactured by Tokyo Chemical Industry Co., Ltd.)

The following was used as a polymerization inhibitor.

4-Methoxyphenol (manufactured by Tokyo Chemical Industry Co., Ltd.)

### Examples 1-1 to 1-4, Comparative Examples 1-1 to 1-2:

### Preparation and Evaluation of Curable Composition Containing Alicyclic Epoxy Acrylate Polymerizable Monomer (Part 1: Combination of Alicyclic Epoxy Acrylate Compound (A) and Thermal Cationic Polymerization Initiator) (1) Preparation of Curable Composition and Cured Product (1-1) Example 1-1

A curable composition was prepared by blending and mixing 45 parts by mass of an epoxy acrylate compound (A1), 45 parts by mass of an epoxy compound (B1), 10 parts by mass of a radical-polymerizable compound (C1), 1 part by mass of a thermal cationic polymerization initiator (D1), and 1 part by mass of a thermal radical polymerization initiator (F1). The obtained curable composition was cured by heating with a hot plate at 80°C for 1 hour, 110°C for 1 hour, 140°C for 1 hour, 180°C for 1 hour, and 240°C for 1 hour to obtain a cured product having a thickness of about 3 mm.

### (1-2) Comparative Examples 1-1 to 1-2, Examples 1-2 to 1-4

A curable composition and a cured product thereof were prepared in the same manner as in Example 1-1 except that the formulation of the curable composition was changed as shown in Table 1-1 below.

**[Table 1-1]**

| | | | Compar ative Example 1-1 | Compar ative Example 1-2 | Exam ple 1-1 | Exam pie 1-2 | Exam pie 1-3 | Exam pie 1-4 |
|---|---|---|---|---|---|---|---|---|
| Epoxy acrylate compound | 4HBAG E | (CA 1) | 45 | | | | | |
| | CM100 | (CA 2) | | 45 | | | | |
| | TCPD-EM | (A1 ) | | | 45 | 45 | 45 | 45 |
| Epoxy compound | CEL20 21P | (B1 ) | 45 | 45 | 45 | 45 | 45 | 45 |
| Radical-polym erizable compound | MMA | (C1 ) | 10 | 10 | 10 | | | |
| | PE-3A | (C3 ) | | | | 10 | | |
| | PE-4A | (C4 ) | | | | | 10 | |
| | DPE-6 A | (C5 ) | | | | | | 10 |
| Thermal cationic polymerization initiator | SI-150 L | (D1 ) | 1 | 1 | 1 | 1 | 1 | 1 |
| Thermal radical polymerization initiator | PERBU TYL O | (F1 ) | 1 | 1 | 1 | 1 | 1 | 1 |

### (2) Evaluation of Physical Properties

### (Weight Loss Temperature of Cured Product of Curable Composition)

The cured products obtained in Examples 1-1 to 1-4 and Comparative Examples 1-1 to 1-2 were each cut into a size of 2 mm in length × 2 mm in width × about 2 mm in thickness to obtain a test piece. The obtained test piece was measured by increasing the temperature from 30°C to 400°C at a rate of 20°C/min using a differential thermal thermogravimetric analysis system (trade name: TG/DTA7300 manufactured by Hitachi High-Tech Corporation). The temperature at which the weight was reduced by 1% compared to the start of measurement was defined as the 1% weight loss temperature. The results are shown in Table 1-2.

### (Heat Resistance of Cured Product of Curable Composition: Tg (DSC))

The glass transition temperature of each cured product test piece having a size of 2 mm in length × 2 mm in width × about 2 mm in thickness obtained as described above was measured by increasing the temperature from 30°C to 400°C at a rate of 10°C/min using a high-sensitivity differential scanning calorimeter (trade name: DSC7000X manufactured by Hitachi High-Tech Corporation). The glass transition temperature as used herein refers to a value measured in accordance with JIS K7121, based on "Midpoint Glass Transition Temperature: T_{mg}" described in the section of "Method for Measuring Transition Temperature of Plastics." The measurement results are shown in Table 1-2.

### (Heat Resistance of Cured Product of Curable Composition: Tg (TMA))

The glass-transition temperature (Tg) (°C) of each of the cured products of the curable compositions obtained in Examples 1-1 to 1-4 and Comparative Examples 1-1 to 1-2 as described above was measured by thermomechanical analysis. Specifically, a test piece having a size of 30 mm in length × 10 mm in width × about 3 mm in thickness was prepared from each of the obtained cured products, and measured by increasing the temperature from 20°C to 300°C at a rate of 10°C/min in a compression mode (98.0665 mN) under nitrogen atmosphere using a thermomechanical analyzer (trade name: TMA7100 manufactured by Hitachi High-Tech Corporation) as a measurement system. The measurement results are shown in Table 1-2.

**[Table 1-2]**

| | | Comparative Example 1-1 | Comparative Example 1-2 | Example 1-1 | Example 1-2 | Example 1-3 | Example 1-4 |
|---|---|---|---|---|---|---|---|
| TG-DTA[°C] | Td1% | 204.2 | 270.2 | 299.4 | 334.2 | 325.2 | 333.5 |
| Tg[°C] | DSC | 112.1 | 113.8 | 229.8 | 240.7 | 209.9 | 219.4 |
| | TMA | 130.5 | 141.9 | 208.1 | 240.0 | 234.0 | 232.2 |

### (Total Luminous Transmittance of Cured Product of Curable Composition)

The total luminous transmittance of each of the cured products (3 mm in thickness) obtained in Examples 1-1 to 1-4 and Comparative Example 1-1 was measured by the method described in JIS K 7631-1 using HAZE METER NDH5000 (manufactured by NIPPON DENSHOKU INDUSTRIES CO., LTD.). The measurement results are shown in Table 1-3.

### (Yellow Index of Cured Product of Curable Composition)

The yellow index (YI) of each of the cured products (3 mm in thickness) obtained in Examples 1-1 to 1-4 and Comparative Example 1-1 was measured by the method described in JIS K7373: 2006 using a spectrophotometer SD6000 (manufactured by NIPPON DENSHOKU INDUSTRIES CO., LTD.). The measurement results are shown in Table 1-3.

**[Table 1-3]**

| | Comparative Example 1-1 | Example 1-1 | Example 1-2 | Example 1-3 | Example 1-4 |
|---|---|---|---|---|---|
| Total luminous transmittance [%] | 49.9 | 52.4 | 70.9 | 73.5 | 73.8 |
| YI | 125.4 | 67.9 | 82.2 | 73.8 | 76.7 |

The values of Tg measured by DSC for the cured products of the curable compositions in Examples 1-1 to 1-4 were 200°C or more, those measured by TMA were 200°C or more, and the 1% thermogravimetric loss temperature was 280°C or more, indicating high heat resistance. Meanwhile, the cured products of the curable compositions in Comparative Examples 1-1 to 1-2 had low heat resistance.

In addition, the total luminous transmittance was 50% or more and the yellow index was 100 or less in Examples 1-1 to 1-4, indicating high transparency. Meanwhile, the cured product of the curable composition in Comparative Example 1-1 had a low degree of transparency.

From the above results, it is understood that the cured product of the curable composition, which is an embodiment of the present invention, is excellent in heat resistance. Further, it is understood that the cured product of the curable composition, which is an embodiment of the present invention, is excellent in transparency.

### Examples 2-1 to 2-5, Comparative Examples 2-1 to 2-3:

### Preparation and Evaluation of Curable Composition Containing Alicyclic Epoxy Acrylate Polymerizable Monomer (Part 2: Combination of Alicyclic Epoxy Acrylate Compound (A) and Curing Agent)

### (1) Preparation of Curable Composition and Cured Product

### (1-1) Example 2-1

A curable composition was prepared by blending and mixing 56 parts by mass of an epoxy acrylate compound (A1), 10 parts by mass of a radical-polymerizable compound (C1), 34 parts by mass of a curing agent (E1), 0.2 parts by mass of a thermal radical polymerization initiator (F2), and 1 part by mass of a curing accelerator (G1), followed by agitation during heating at 50°C by a hot plate. The obtained curable composition was cured by heating with a hot plate at 70°C for 1 hour, 140°C for 1 hour, 190°C for 2 hours, and 240°C for 4 hours to obtain a cured product having a thickness of about 3 mm.

### (1-2) Examples 2-2 to 2-5

A curable composition and a cured product thereof were prepared in the same manner as in Example 2-1 except that the formulation of the curable composition was changed as shown in Table 2 below.

### (1-3) Comparative Examples 2-1 to 2-3

A curable composition and a cured product thereof were prepared in the same manner as in Example 2-1 except that the formulation of the curable composition was changed as shown in Table 2-1 below and mixing was performed at room temperature instead of agitation during heating at 50°C by a hot plate.

**[Table 2-1]**

| | | | Compa rative Exampl e 2-1 | Compa rative Exampl e 2-2 | Compa rative Exampl e 2-3 | Exa mple 2-1 | Exa mple 2-2 | Exa mple 2-3 | Exa mple 2-4 | Exa mple 2-5 |
|---|---|---|---|---|---|---|---|---|---|---|
| Epoxy acrylate compound | 4HBAGE | (CA1) | 47 | | | | | | | |
| | CM100 | (CA2) (B2) | | 47 | | | 25.6 | 25.6 | 25.6 | 25.6 |
| | GMA | (CA3) | | | 39 | | | | | |
| | TCPD-EM | (A1) | | | | 56 | 25.6 | 25.6 | 25.6 | 25.6 |
| Radical-pol ymerizable compound | MMA | (C1) | 10 | 10 | 10 | 10 | 10 | | | |
| | PE-3A | (C3) | | | | | | 10 | | |
| | PE-4A | (G4) | | | | | | | 10 | |
| | DPE-6A | (C5) | | | | | | | | 10 |
| Curing agent | HN-5500 | (EI) | 43 | 43 | 51 | 34 | 38.8 | 38.8 | 38.8 | 38.8 |
| Thermal radical polymeriza tion initiator | AIBN | (F2) | 0.2 | 0.2 | 0.2 | 0.2 | 0.2 | 0.2 | 0.2 | 0.2 |
| Curing accelerator | PX-4ET | (G1) | 1 | 1 | 1 | 1 | 1 | 1 | 1 | 1 |

### (2) Evaluation of Physical Properties

### (Weight Loss Temperature of Cured Product of Curable Composition)

The weight loss temperature of each of the cured products obtained in the Examples and the Comparative Examples was measured. Specifically, the weight loss temperature of the cured product was measured in the same manner as in Example 1 except that the measurement temperature was the temperature at which the weight of the test piece was reduced by 5% compared to the start of measurement. The measurement results are shown in Table 2-2.

### (Heat Resistance of Cured Product of Curable Composition: Tg (DSC))

Heat resistance of each of the cured products obtained in the Examples and the Comparative Examples was measured in the same manner as in Example 1. The measurement results are shown in Table 2-2.

**[Table 2-2]**

| | | Comparati ve Example 2-1 | Comparati ve Example 2-2 | Comparati ve Example 2-3 | Exampl e 2-1 | Exampl e 2-2 | Exampl e 2-3 | Exampl e 2-4 | Exampl e 2-5 |
|---|---|---|---|---|---|---|---|---|---|
| TG-DTA [°C] | Td5 % | 310.0 | 312.1 | 290.5 | 330.6 | 323.7 | 325.1 | 323.2 | 322.6 |
| Tg[°C] | DS C | 56.0 | 227.2 | 138.0 | 270.2 | 231.0 | 252.5 | 252.8 | 256.3 |

### (Total Luminous Transmittance of Cured Product of Curable Composition)

Total luminous transmittance of each of the cured products obtained in Examples 2-2 to 2-5 and Comparative Example 2-3 was measured in the same manner as in Example 1. The measurement results are shown in Table 2-3.

### (Yellow Index of Cured Product of Curable Composition)

The yellow index of each of the cured products obtained in Examples 2-2 to 2-5 and Comparative Example 2-3 was measured in the same manner as in Example 1. The measurement results are shown in Table 2-3.

**[Table 2-3]**

| | Comparative Example 2-3 | Example 2-2 | Example 2-3 | Example 2-4 | Example 2-5 |
|---|---|---|---|---|---|
| Total luminous transmittance [%] | 45.9 | 72.5 | 73.8 | 76.7 | 68.7 |
| YI | 85.6 | 38.8 | 56.3 | 55.6 | 66.8 |

The values of Tg measured by DSC for the cured products of the curable compositions in Examples 2-1 to 2-5 were 230°C or more, and the 5% thermogravimetric loss temperature was 320°C or more, indicating high heat resistance. Meanwhile, the cured products of the curable compositions in Comparative Examples 2-1 to 2-3 had low heat resistance.

In addition, the total luminous transmittance was 50% or more and the yellow index was 50 or less in Example 2-2, indicating high transparency. The total luminous transmittance was 50% or more and the yellow index was 70 or less in Examples 2-3 to 2-5, indicating high transparency. Meanwhile, the cured product of the curable composition in Comparative Example 2-3 had a low degree of transparency.

From the above results, it is understood that the cured product of the curable composition, which is an embodiment of the present invention, is excellent in heat resistance. Further, it is understood that the cured product of the curable composition, which is an embodiment of the present invention, is excellent in transparency.

### Example 3-1, Comparative Examples 3-1 to 3-3: Preparation and Evaluation of Curable Composition Containing Alicyclic Epoxy Acrylate Polymerizable Monomer (Part 3: Combination of Epoxy Group-Containing (Meth)acrylic Polymer and Photo-cationic Polymerization Initiator)

### (1) Preparation of Epoxy Group-Containing] (Meth)acrylic Polymer

### (1-1) Example 3-1

Into a reaction vessel equipped with a thermometer, an agitator, and a reflux tube, 23 parts by mass of an epoxy acrylate compound (A1), 77 parts by mass of an epoxy compound (B1), and 3 parts by mass of a thermal radical polymerization initiator (F1) were added, and the mixture was agitated at 200 rpm for 3 hours during heating in an oil bath at 100°C. Here, the epoxy compound (B1) did not undergo a polymerization reaction. Then, the mixture was allowed to cool to room temperature to obtain an epoxy group-containing (meth)acrylic polymer. Therefore, the epoxy group-containing (meth)acrylic polymer coexisted with the epoxy compound (B1) (hereinafter, also referred to as "mixture (J1) of the epoxy group-containing (meth)acrylic polymer and the epoxy compound (B1)).

### (1-2) Comparative Examples 3-1 to 3-3

A mixture of an epoxy group-containing (meth)acrylic polymer and an epoxy compound was obtained in the same manner as in Example 3-1 except that the formulation was changed as shown in Table 3 below.

### (2) Evaluation

### (Number Average Molecular Weight and Weight Average Molecular Weight of Epoxy Group-Contain (Meth)acrylic Polymer)

The number average molecular weight(Mn) and the weight average molecular weight (Mw) were measured by GPC (gel permeation chromatography) for the epoxy group-containing (meth)acrylic polymers obtained in Example 3-1 and Comparative Examples 3-1 to 3-3. Specifically, the measurement was carried out under the following conditions.

### [GPC Conditions]

Measurement system: HLC-8220GPC (Tosoh Corporation) Column: 1 column of TOSOH TSK guard column Super MP (HZ)-M (Tosoh Corporation), 3 columns of TOSOH TSK gel SuperMultipore HZ-M (1 column of TOSOH TSK guard column was followed by 3 columns of TOSOH TSK gel SuperMultipore HZ-M connected in series after.)
Flow rate: 0.35 mL/min
Developing solvent: Tetrahydrofuran
Injection volume: 25 µL (tetrahydrofuran solution with a sample concentration of 5% by mass)
Column temperature: 25°C
Detector: UV
Detection wavelength: 254 nm
Reference standard: The following monodispersed polystyrene having a known molecular weight was used.
(Polystyrene used)
"PstQuick MP-M" (Tosoh Corporation)

The measurement results of Mn and Mw for the obtained epoxy group-containing (meth)acrylic polymers are shown in Table 3. Note that the epoxy compound remained in the monomer form in the mixture of the epoxy group-containing (meth)acrylic polymer and the epoxy compound. Therefore, regarding Mn and Mw, from the results obtained by GPC, only measurement results of the high molecular weight peak from the epoxy group-containing (meth)acrylic polymer are shown.

### (Infrared Absorption Spectrum of Epoxy Group-Containing (Meth)acrylic Polymer)

The infrared absorption spectrum (IR) of the mixture of the epoxy group-containing (meth)acrylic polymer and the epoxy compound obtained in Example 3-1 was measured under the following conditions by the ATR method
System: Nicolet iS10 (Thermo Fisher Scientific)
Measurement range: 4000 to 400 cm⁻¹
Number of accumulation times: 32 times

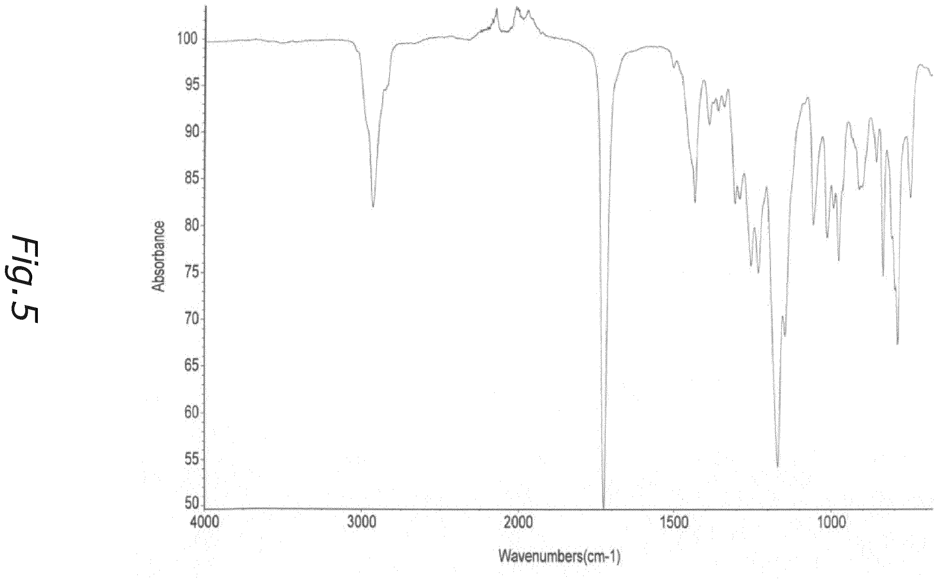

FIG. 5 shows the obtained infrared absorption spectrum. In the IR of the acrylic compound, there is a double-bond-derived peak usually in the vicinity of 1640 cm⁻¹. However, in FIG. 5, only the largest peak is confirmed at 1710 cm⁻¹, and no double-bond-derived peak can be confirmed. Therefore, it was confirmed that the progression of radical polymerization eliminated the double bond of acrylic, and thus the desired epoxy group-containing (meth)acrylic polymer represented by Formula (2) was formed.

**[Table 3]**

| | | | | Comparative Example 3-1 | Comparative Example 3-2 | Comparative Example 3-3 | Example 3-1 |
|---|---|---|---|---|---|---|---|
| | | | | Mixture CJ1 | Mixture CJ2 | Mixture CJ3 | Mixture J1 |
| Epoxy acrylate compound | 4HBAGE | (GA1) | [Part(s) by mass] | 23 | | | |
| | CM100 | (GA2) | | | 23 | | |
| | GMA | (GA3) | | | | 23 | |
| | TCPD-EM | (A1) | | | | | 23 |
| Epoxy compound | CEL2021P | (B1) | | 77 | 77 | 77 | 77 |
| Thermal radical polymerization initiator | PERBUTYL O | (F1) | | 3 | 3 | 4 | 3 |
| Mn | | | | 5,472 | 6,335 | 5,791 | 30,515 |
| Mw | | - | | 31,485 | 29,576 | 25,507 | 84,494 |

### (3) Preparation of Curable Composition and Cured Product

### (3-1) Example 3-1

A curable composition was prepared by blending and mixing 100 parts by mass of the mixture (J1) of the epoxy group-containing (meth)acrylic polymer and the epoxy compound prepared in (1-1) with 1 part by mass of a photo-cationic polymerization initiator (H1). The obtained curable composition was charged into an aluminum cup and irradiated with UV light at a cumulative irradiation dose described in Table 4 at room temperature to cure the curable composition, thereby obtaining a cured product having a thickness of 3 mm.

### (3-2) Comparative Examples 3-1 to 3-3

A curable composition and a cured product thereof were prepared in the same manner as in Example 3-1 except that the formulation of the curable composition and the cumulative irradiation dose of UV light were changed as shown in Table 4 below.

### (4) Evaluation of Physical Properties

### (Weight Loss Temperature of Cured Product of Curable Composition)

The weight loss temperature of each of the cured products obtained in the Examples and the Comparative Examples was measured. Specifically, the weight loss temperature of the cured product was measured in the same manner as in Example 1 except that the measurement temperature was the temperature at which the weight of the test piece was reduced by 5% or 10% compared to the start of measurement. The measurement results are shown in Table 4.

**[Table 4]**

| | | | | Comparative Example 3-1 | Comparative Example 3-2 | Comparative Example 3-3 | Example 3-1 |
|---|---|---|---|---|---|---|---|
| Mixture of epoxy group-containing (meth)acrylic polymer and epoxy compound | Mixture CJ1 | (CJ1) | [Part(s) by mass] | 100 | | | |
| | Mixture CJ2 | (CJ2) | | | 100 | | |
| | Mixture CJ3 | (CJ3) | | | | 100 | |
| | Mixture J1 | (J1) | | | | | 100 |
| Photo-cationlc polymerization initiator | CPI-200K | (H1) | | 1 | 1 | 1 | 1 |
| Cumulative irradiation dose | | | mJ/cm² | 569 | 1038 | 3156 | 554 |
| Weight loss | Td5% | | [°C] | 281.25 | 278.4 | 263.8 | 299.75 |
| | Td10% | | | 298.25 | 308.5 | 290.25 | 324.85 |

The 5% thermogravimetric loss temperature was 290°C or more and the 10% thermogravimetric loss temperature was 320°C or more in Example 3-1, indicating high heat resistance. Meanwhile, the cured products of the curable compositions in Comparative Examples 3-1 to 3-3 had low heat resistance.

From the above results, it is understood that the cured product of the curable composition, which is an embodiment of the present invention, is excellent in heat resistance.

### Examples to 3-2. Comparative Examples 3-4 to 3-5: Preparation and Evaluation of Curable Composition Containing Alicyclic Epoxy Acrylate Polymerizable Monomer (Part 4: Combination of Alicyclic Epoxy Acrylate Compound (A) and Photo-Cationic Polymerization Initiator)

### (1) Preparation of Curable Composition and Cured Product

### (1-1) Example 3-2

A curable composition was obtained by blending and mixing 33 parts by mass of the epoxy acrylate compound (A1), 67 parts by mass of the epoxy compound (B1), 3 parts by mass of the photo-cationic polymerization initiator (H2), and 1 part by mass of a photosensitizer. The obtained curable composition was poured into charged into a 1.5-cm square silicone frame and irradiated with UV light at a cumulative irradiation dose of 2924 mJ/cm² 4 at room temperature to cure the curable composition, thereby obtaining a cured product having a thickness of 1 mm.

### (1-2) Comparative Examples 3-4 to 3-5

A curable composition and a cured product thereof were prepared in the same manner as in Example 3-2 except that the formulation of the curable composition was changed as shown in Table 5 below.

### (2) Evaluation of Physical Properties

### (Weight Loss Temperature of Cured Product of Curable Composition)

The weight loss temperature of each of the cured products obtained in the Examples and the Comparative Examples was measured. Specifically, the weight loss temperature of the cured product was measured in the same manner as in Example 1 except that the measurement temperature was the temperature at which the weight of the test piece was reduced by 5% or 10% compared to the start of measurement. The measurement results are shown in Table 5.

**[Table 5]**

| | | | | Comparative Example 3-4 | Comparative Example 3-5 | Example 3-2 |
|---|---|---|---|---|---|---|
| Epoxy acrylate | 4HBAGE | (CA1) | [Part(s) by | 33 | | |
| compound | CM100 | (CA2) | mass] | | 33 | |
| | TCPD-EM | (A1) | | | | 33 |
| Epoxy compound | CEL2021P | (B1) | | 67 | 67 | 67 |
| Photo-cationic polymerization initiator | CPI-210S | (H2) | | 3 | 3 | 3 |
| Photosensitizer | DETX-S | | | 1 | 1 | 1 |
| Weight loss | Td5% | | [°C] | 232 | 233 | 240 |
| | Td10% | | | 261 | 257 | 269 |

The 5% thermogravimetric loss temperature was 235°C or more and the 10% thermogravimetric loss temperature was 265°C or more in Example 3-2, indicating high heat resistance. Meanwhile, the cured products of the curable compositions in Comparative Examples 3-4 to 3-5 had low heat resistance.

From the above results, it is understood that the cured product of the curable composition, which is an embodiment of the present invention, is excellent in heat resistance.

### Example 4-1, Comparative Examples 4-1 to 4-3: Preparation and Evaluation of Curable Composition Containing Alicyclic Epoxy Acrylate Polymerizable Monomer (Part 5: Combination of Radical-polymerizable Compound and Photoradical Polymerization Initiator)

### (1) Production of Trunk Polymer (Example 4-1, Comparative Examples 4-1 to 4-3)

Into a reaction vessel equipped with a thermometer, an agitator, and a reflux tube, 50 g of dipropyleneglycol monomethyl ether (MFDG)(manufactured by Tokyo Chemical Industry Co., Ltd.) was added and heated to 110°C. In addition, a solution was prepared separately by mixing 32 g of methyl methacrylate (C1), 30 g of acrylic acid (C2), 46 g of dipropyleneglycol monomethyl ether, and 4.7 g of t-butylperoxy-2-ethylhexanoate (PERBUTYL (registered trademark) O) (F1), and this was added dropwise over 1 hour to the reaction vessel. After completion of the dropwise addition, agitation was continued at 110°C for 3 hours. Thereafter, it was allowed to cool to room temperature to obtain 162 g of a trunk polymer.

### (2) Preparation of Radical-polymerizable Compound (K)

### (2-1) Example 4-1

Into a reaction vessel equipped with a thermometer, an agitator, and a reflux tube, 20 g of the trunk polymer obtained in (1) above, 15.3 g of the epoxy acrylate compound (A1), a catalyst (Pph3) at 1 mol% (based on the amount of substance of the epoxy acrylate compound), polymerization inhibitor (hydroquinone monomethylether) at 0.5 mol% (based on the amount of substance of the epoxy acrylate compound) were added and agitated at 200 rpm in an oil bath at 100°C for 8 hours. Thereafter, it was allowed to cool to room temperature to obtain a radical-polymerizable compound (K1).

The number average molecular weight (Mn) and the weight average molecular weight (Mw) were measured in the same manner as in Example 3-1 for the obtained radical-polymerizable compound (K1). The obtained measurement results are shown in Table 6.

### (2-2) Comparative Examples 4-1 to 4-3

A radical-polymerizable compound was obtained in the same manner as in Example 4-1 except that the formulation was changed as shown in Table 6 below.

### (3) Evaluation

### (Number Average Molecular Weight and Weight Average Molecular Weight of Radical-polymerizable Compound)

The number average molecular weight (Mn) and the weight average molecular weight (Mw) were measured in the same manner as in Example 3-1 for the radical-polymerizable compounds obtained in Example 4-1 and Comparative Examples 4-1 to 4-3.

### (Infrared Absorption Spectrum of Radical-polvmerizable Compound (K1))

(2-1) The infrared absorption spectrum (IR) was measured in the same manner as in Example 3-1 for the radical-polymerizable compound (K1) obtained in Example 4-1,

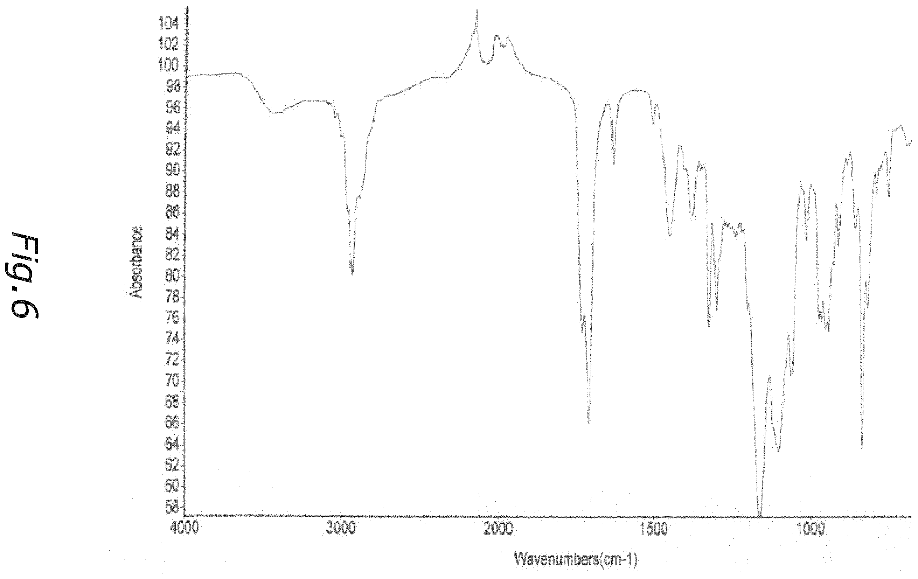

(2-1) FIG. 6 shows the infrared absorption spectrum of the radical-polymerizable compound (K1) obtained in Example 4-1. In FIG. 6, two kinds of functional groups which are an OH group (the broad peak from 3400 to 3500 cm⁻¹) and an acrylic moiety (1640 cm⁻¹) could be confirmed. Therefore, it was confirmed that the desired radical-polymerizable compound represented by Formula (3) was formed.

**[Table 6]**

| Preparation of radical polymerizable compound | | | | | | | |
|---|---|---|---|---|---|---|---|
| | | | | Comparativ e Example 4-1 | Comparative Example 4-2 | Comparative Example 4-3 | Example 4-1 |
| | | | | Polymerizab le compound CK1 | Polymerizable compound CK2 | Polymerizable compound CK3 | Polymerizabl e compound KI |
| Trunk polymer | MMA/AA copolymer | | [g] | 20 | 20 | 20 | 20 |
| Epoxy acrylate compound | 4HBAGE | (CA1) | [g] ^{∗}1 | 10.2 | | | |
| | CM100 | (CA2) | | | 10 | | |
| | GMA | (CA3) | | | | 7.7 | |
| | TCPD-EM | (A1) | | | | | 15.3 |
| Catalyst | PPh3 | | [mol%] ^{∗}2 | 1 | 1 | 1 | 1 |
| Polymerizat ion inhibitor | 4-Methoxy phenol | | [mel %] ^{∗}2 | 0.5 | 0.5 | 0.5 | 0.5 |
| Molecular weight | Mn | | | 8024 | 7537 | 7367 | 5415 |
| | Mw | | | 17022 | 14049 | 17573 | 9599 |

| | | | | | | | |
|---|---|---|---|---|---|---|---|
| ^{∗}1: The amount of substance was the same as that of carboxylic acid (51 mmol) in the trunk polymer. ^{∗}2: The amount of substance of the epoxy acrylate compound was used as a reference. | | | | | | | |

### (4) Preparation of Curable Composition and Cured Product

### (4-1) Example 4-1

A curable composition was prepared by blending and mixing 67 parts by mass of the radical-polymerizable compound (K1) prepared in (2), 33 parts by mass of a radical-polymerizable compound (C3) different from the radical-polymerizable compound (K1), and 1 part by mass of a photoradical polymerization initiator (I1). The obtained curable composition was charged into an aluminum cup and irradiated with UV light at a cumulative irradiation dose described in Table 7 at room temperature to cure the curable composition, thereby obtaining a cured product having a thickness of 3 mm.

### (4-2) Comparative Examples 4-1 to 4-3

A curable composition and a cured product thereof were prepared in the same manner as in Example 4-1 except that the formulation of the curable composition and the cumulative irradiation dose of UV light were changed as shown in Table 7-1 below.

### (5-1) Evaluation of Physical Properties of Cured Products Described in Example 4-1, Comparative Examples 4-1 to 4-3 (Weight Loss Temperature of Cured Product of Curable Composition)

The weight loss temperature of each of the cured products obtained in the Examples and the Comparative Examples was measured. Specifically, the weight loss temperature of the cured product was measured in the same manner as in Example 1 except that the measurement temperature was the temperature at which the weight of the test piece was reduced by 1%, 5%, or 10% compared to the start of measurement. The measurement results are shown in Table 7-1.

### (Heat Resistance of Cured Product of Curable Composition: Tg (TMA))

Heat resistance of each of the cured products obtained in the Examples and the Comparative Examples was measured in the same manner as in Example 1. The measurement results are shown in Table 7-1.

**[Table 7-1]**

| UV radical electrification | | | | | | | |
|---|---|---|---|---|---|---|---|
| | | | | Comparative Example 4-1 | Comparative Example 4-2 | Comparative Example 4-3 | Example 4-1 |
| Radical-polymerizable compound | Polymerizable compound CK1 | (CK1) | [Part(s) by mass] | 67 | | | |
| | Polymerizable compound CK2 | (CK2) | | | 67 | | |
| | Polymerizable compound CK3 | (CK3) | | | | 67 | |
| | Polymerizable compound KI | (K1) | | | | | 67 |
| Radical-polymerizable compound | Light acrylate PE-3A | (C3) | | 33 | 33 | 33 | 33 |
| Photoradical polymerization initiator | MPBB | (I1) | | 1 | 1 | 1 | 1 |
| Cumulative irradiation dose | | | mJ/cm² | 452 | 452 | 3247 | 616 |
| Weight loss | Td1% | | [°C] | 68.2 | 58.3 | 64.3 | 93.3 |
| | Td5% | | | 114.8 | 105.4 | 116.4 | 166.9 |
| | Td10% | | | 152.3 | 139.7 | 170.4 | 204.5 |
| Glass-transition temperature | Tg (TMA, compression) | | [°C] | 59.2 | 95.4 | 68.4 | 136.8 |

The 1% thermogravimetric loss temperature was 70°C or more, the 5% thermogravimetric loss temperature was 150°C or more, the 10% thermogravimetric loss temperature was 180°C or more, and the Tg measured by TMA was 100°C or more in Example 4-1, indicating high heat resistance. Meanwhile, the cured products of the curable compositions in Comparative Examples 4-1 to 4-3 had low heat resistance.

From the above results, it is understood that the cured product of the curable composition, which is an embodiment of the present invention, is excellent in heat resistance.

### (4-3) Examples 4-2 to 4-3

A curable composition and a cured product thereof were prepared in the same manner as in Example 4-1 except that the formulation of the curable composition and the cumulative irradiation dose of UV light were changed as shown in Table 7-2 below.

### (5-2) Evaluation of Physical Properties of Cured Products Described in Examples 4-2 to 4-3

### (Weight Loss Temperature of Cured Product of Curable Composition)

The weight loss temperature of each of the cured products obtained in Examples 4-2 to 4-3 was measured. Specifically, the weight loss temperature of the cured product was measured in the same manner as in Example 1 except that the measurement temperature was the temperature at which the weight of the test piece was reduced by 1%, 5%, or 10% compared to the start of measurement. The measurement results are shown in Table 7-2.

**[Table 7-2]**

| UV radical curing | | | | | |
|---|---|---|---|---|---|
| | | | | Example 4-2 | Example 4-3 |
| Radical-polymerizable compound | Polymerizable compound K1 | (K1) | [Part(s) by mass] | 67 | 67 |
| Radical-polymerizable compound | PE-14A | (C4) | | 33 | |
| | DPIE-6A | (C5) | | | 33 |
| Photoradical polymerization initiator | MPBB | (I1) | | 1 | 1 |
| Cumulative irradiation dose | | | mJ/cm² | 1155 | 1155 |
| Weight loss | Td1% | | [°C] | 106.1 | 105.1 |
| | Td5% | | | 188.0 | 182.8 |
| | Td10% | | | 243.9 | 241.9 |

The 1% thermogravimetric loss temperature was 70°C or more, the 5% thermogravimetric loss temperature was 150°C or more, and the 10% thermogravimetric loss temperature was 180°C or more in Examples 4-2 to 4-3, indicating higher heat resistance as compared to the above-described cured products of the curable compositions in Comparative Examples 4-1 to 4-3.
From the above results, it is understood that the cured product of the curable composition, which is an embodiment of the present invention, is excellent in heat resistance.

## Claims

1. An alicyclic acrylate compound represented by the following Formula (1): 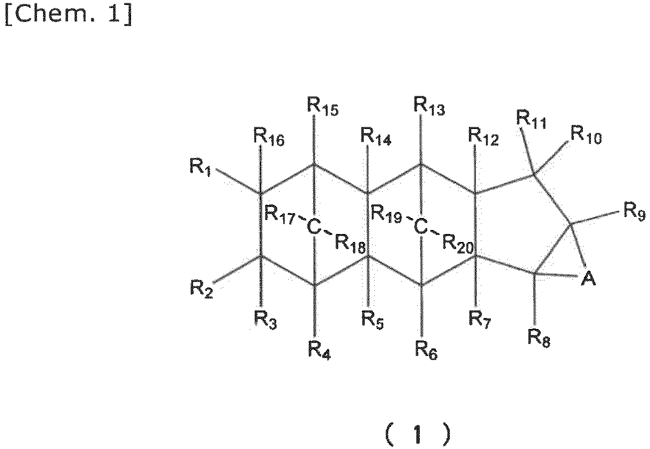 wherein one of R₁ and R₂ is a (meth)acryloyloxy group,
the other of R₁ and R₂ is a hydrogen atom,
R₃ to R₂₀ are each independently selected from the group consisting of a hydrogen atom, an alkyl group, and an alkoxy group, and
A is an oxygen atom or A is not present, and a carbon atom to which R₈ binds and a carbon atom to which R₉ binds together form a double bond.

2. An alicyclic acrylate-type polymerizable monomer comprising the alicyclic acrylate compound according to claim 1.

3. The alicyclic acrylate compound according to claim 1, which is an alicyclic epoxy acrylate compound represented by the Formula (1), wherein A is an oxygen atom.

4. An alicyclic epoxy acrylate-type polymerizable monomer comprising the alicyclic epoxy acrylate compound according to claim 3.

5. A curable composition comprising at least the alicyclic epoxy acrylate-type polymerizable monomer according to claim 4.

6. The curable composition according to claim 5, which further comprises at least one selected from the group consisting of a curing agent, a thermal cationic polymerization initiator, and a photo-cationic polymerization initiator.

7. The curable composition according to claim 6, wherein the curing agent is an acid anhydride-based compound.

8. The curable composition according to any one of claims 5 to 7, which further comprises a thermal radical polymerization initiator.

9. The curable composition according to any one of claims 5 to 8, which further comprises an epoxy compound that is different from the alicyclic epoxy acrylate compound.

10. The curable composition according to any one of claims 5 to 9, which further comprises a radical-polymerizable compound that is different from the alicyclic epoxy acrylate compound.

11. An epoxy group-containing (meth)acrylic polymer comprising at least a polymerization unit represented by the following Formula (2): 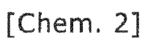 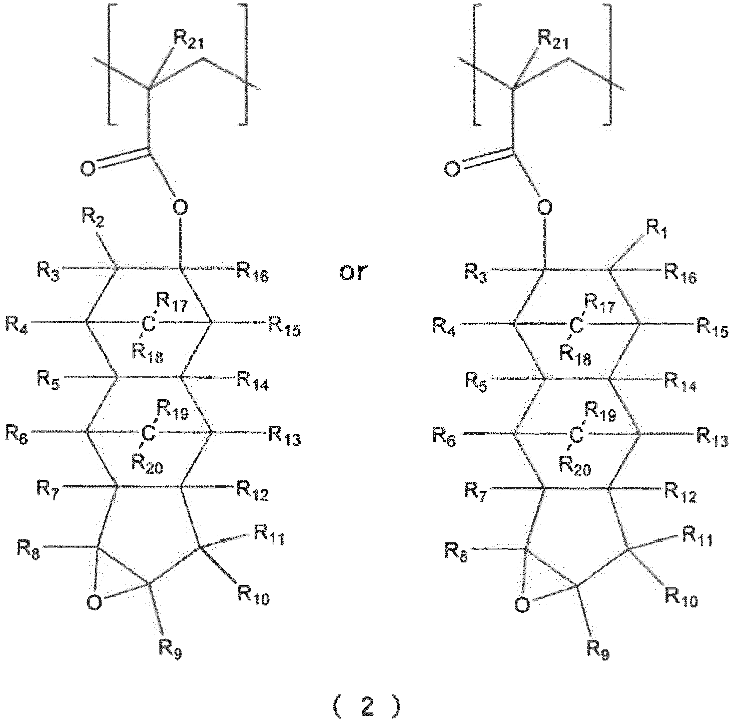 wherein R₁ and R₂ are each a hydrogen atom,
R₃ to R₂₀ are each independently selected from the group consisting of a hydrogen atom, an alkyl group, and an alkoxy group, and
R₂₁ is a hydrogen atom or a methyl group.

12. A curable composition comprising at least the epoxy group-containing (meth)acrylic polymer according to claim 11.

13. The curable composition according to claim 12, which further comprises a photo-cationic polymerization initiator.

14. The curable composition according to claim 12 or 13, which further comprises an epoxy compound that is different from the epoxy group-containing (meth)acrylic polymer.

15. A radical-polymerizable compound comprising at least a polymerization unit represented by the following Formula (3): 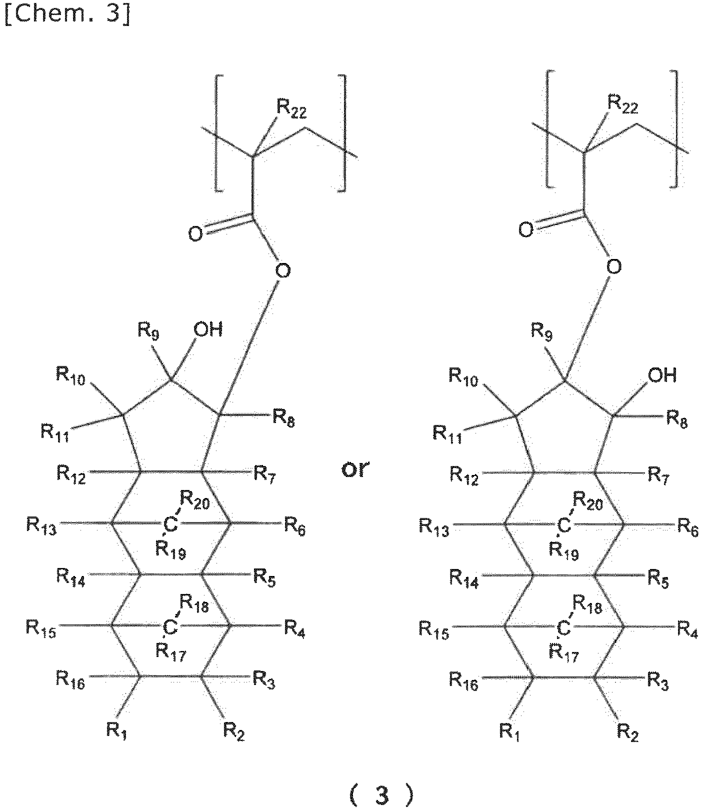 wherein one of R₁ and R₂ is a (meth)acryloyloxy group,
the other of R₁ and R₂ is a hydrogen atom,
R₃ to R₂₀ are each independently selected from the group consisting of a hydrogen atom, an alkyl group, and an alkoxy group, and
R₂₂ is selected from a hydrogen atom and a methyl group.

16. The radical-polymerizable compound according to claim 15, which further comprises a polymerization unit derived from at least one acrylic compound selected from (meth)acrylic acid ester and (meth)acrylic acid.

17. A curable composition comprising at least the radical-polymerizable compound according to claim 15 or 16.

18. The curable composition according to claim 17, which further comprises a photoradical polymerization initiator.

19. The curable composition according to claim 17 or 18, which further comprises a radical-polymerizable compound that is different from the radical-polymerizable compound.

20. A method of producing an epoxy group-containing acrylic polymer, comprising a step of radically polymerizing the alicyclic epoxy acrylate compound according to claim 3.

21. A method of producing a radical-polymerizable compound, comprising a step of reacting a polymer of at least one acrylic compound selected from (meth)acrylic acid ester and (meth)acrylic acid with the alicyclic epoxy acrylate compound according to claim 3.
